# EUROPÄISCHE PATENTANMELDUNG

(11) **EP 4 187 458 A1**
(43) Veröffentlichungstag der Anmeldung: **31.05.2023**
(21) Anmeldenummer: 21210389.9
(22) Anmeldetag: 25.11.2021
(51) Int. Cl.: G06Q 10/08, G06Q 10/10, G16C 99/00

(54) **VERFAHREN ZUR ELEKTRONISCHEN AUFZEICHNUNG VON EXPERIMENTEN**

(71) Anmelder: Covestro Deutschland AG, 51373 Leverkusen (DE)
(72) Erfinder: FÄCKE, Thomas, 51375 Leverkusen (DE); SCHOLZ, Stephan, 51373 Leverkusen (DE); CASPERS, Lennart, 51373 Leverkusen (DE); BACH, Johannes, 51373 Leverkusen (DE)
(74) Vertreter: Levpat

(57) **Zusammenfassung**

Die Erfindung betrifft ein computerimplementiertes Verfahren zur elektronischen Aufzeichnung von naturwissenschaftlichen Experimenten in einem elektronischen Laborjournal, welches durch folgende Verfahrensschritte gekennzeichnet ist:
- Bereitstellung wenigstens einer Chemikalie sowie wenigstens eines Laborobjektes und/oder wenigstens eines Labormessgerätes zur Durchführung eines naturwissenschaftlichen Experimentes,
- Registrierung der wenigstens einen Chemikalie sowie des wenigstens einen Laborobjektes und/oder des wenigstens einen Labormessgerätes zumindest für die Zeit ihrer/seiner Verwendung in dem naturwissenschaftlichen Experiment und Verknüpfung mit einer Laborjournal-Experimentkennung jeweils in einem Computersystem,
- Übermittlung wenigstens eines Ereignisses und/oder wenigstens einer Beobachtung zu der wenigstens einen Chemikalie und /oder zu dem wenigstens einen Laborobjekt und/oder wenigstens eines Messergebnisses des wenigstens einen Labormessgerätes an das Computersystem jeweils zusammen mit einem Zeitstempel unter Anwendung der Verknüpfung mit der Laborjournal-Experimentkennung und optional weiterer Verknüpfungsregeln, wobei das Computersystem wenigstens eine Daten-Reihe erzeugt und in einem elektronischen Laborjournal abspeichert.

Ferner betrifft die vorliegende Erfindung ein System

## Beschreibung

Die Erfindung betrifft ein Verfahren zur elektronischen Aufzeichnung von naturwissenschaftlich-technischer Versuchstätigkeit, insbesondere von naturwissenschaftlichen Experimenten. Ferner betrifft die vorliegenden Erfindung ein System zur elektronischen Aufzeichnung von naturwissenschaftlich-technischer Versuchstätigkeit, insbesondere von naturwissenschaftlichen Experimenten.

Die praktische Durchführung von Experimenten in einem Labor umfasst eine Vielzahl von verschiedenen Arbeitsschritten. Eine exakte Protokollierung der Vorgänge in einem Experiment zum Zweck der Wissensvermehrung wird bereits in der Schule, in der Ausbildung und umso mehr in der universitären Lehre vermittelt. Die hiermit erstellten Protokolle dienen dabei einer möglichst objektiven und nacharbeitbaren Dokumentation. Die wissenschaftliche Erkenntnis in empirischen Wissenschaften, wie den Naturwissenschaften und Sozialwissenschaften, wird maßgeblich von der Überprüfung von Hypothesen an solchen Dokumentationen vorangetrieben. Auch Wirtschaftsunternehmen folgen dieser Arbeitsweise, wenn auch der Zweck i.d.R. nicht allein dem Wissensgewinn, sondern insbesondere der Steigerung der Wettbewerbsfähigkeit dient. So werden in der Durchführung von weiteren Experimenten neue Produkte hergestellt oder geprüft, Verfahren optimiert oder neue Erkenntnisse gewonnen, die es schließlich erlauben neue, bessere, kostengünstigere Produkte oder Leistungen anbieten zu können.

In vielen Einzelwissenschaften haben sich bestimmte Prozeduren, Verfahren und Meßmethoden gemäß dem Wissensstand meist auch angepasst an übliche Industriepraktiken etabliert, die die übliche Durchführung von Experimenten bestimmen. Es sind oft die Verfahrensparameter, Rezepturen, Eingangstoffe, die in solchen Experimenten variiert bzw. ausgetauscht werden. Eine gängige Vorgehensweise dabei ist die systematische Veränderung solcher Experimentbedingungen, die eine Optimierung erlauben. Obwohl dieses Vorgehen suggeriert, dass die tägliche Praxis in einem Forschungs- und Entwicklungslabor vorhersagbar ist, gibt es tatsächlich aber tagtäglich unerwartete Beobachtungen und adaptierte Experimentdurchführungen, die manchmal zu größerem Wissensgewin führen oder leider nur zu oft die Reproduktions falsifizieren oder sogar die geplante Durchführung umfänglich verhindern.

In einer Wirtschaftswelt, die sich durch Arbeitsteilung optimiert hat, ist es zudem häufige Praxis, dass der Planer von Experimenten und der Durchführende (der Experimentator) nicht dieselben Personen sind. Eine gute Dokumentation der Experimente ist daher essentiell, stößt aber auf grundlegende Probleme durch die individuelle Vorerfahrung dieser Handelnden. Da die Dokumentation mit Aufwand verbunden ist, folgt der Experimentator seiner eigenen, subjektiven Einschätzung was dokumentationswürdig erscheint und dokumentiert die für ihn vermeintlichen Selbstverständlichkeiten nur rudimentär. Erkenntnisse werden oft zwischen Planendem und dem Experimentator auf direktem Wege ausgetauscht und sind in der Experimentdokumentation nicht oder nur unvollständig enthalten. Noch schwieriger ist die nachträgliche Auswertung einer derartigen Dokumentation von Dritten in der Organisation, wenn diese Dokumentation nachträglich allen Interessierten in der Organisation zur Verfügung gestellt werden soll. Diese Dritten waren in den Kontext der Versuche meist nur peripher involviert und die Dokumentation ist damit aus dem Kontext der Kommunikation zwischen Planer und Experimentator gerissen, wirkt fragmentiert und daher unverständlich. Da die Versuchsdurchführung aufwändig, also teuer ist, besteht somit ein großer Bedarf an einer besseren, vergleichbaren Dokumentation von Experimenten.

Obwohl handschriftliche Protokolle noch vereinzelt praktiziert werden, wurde mit der breiten Verfügbarkeit von elektronischen Medien seit den 90er Jahren die Dokumentation experimenteller Arbeit in vielen Industrien umgestellt. Dabei gibt es heute verschiedene Implementierungsformen, so z.B. die Ablage in Texteditoren, Tabellenkalkulationen oder auch auf Datenbank basierenden Programme, die die Dokumentation in vordefinierten Feldern oder Workflows ermöglichen. In anwendungsspezifischen Software-Implementierungen, z.B. für die Materialindustrie, werden dabei Formulierungen und analytische Daten abgelegt. Zudem werden Prozessinformationen, Planungsgrundlagen, Projektziele und andere Metadaten in Textform i.d.R. nur schwer oder gar nicht recherchierbar abgelegt.

Derartige Daten sind selten konsistent abgelegt, da es den Experimentatoren schwerfällt und/oder einen erheblichen, teilweise nicht leistbaren zeitlichen Aufwand darstellt, Daten systematisch aufzubereiten und abzuspeichern. Für moderne Auswerteverfahren mittels Algorithmen und Verfahren der künstlichen Intelligenz benötigt man allerdings "kurierte" Daten, die vergleichbar erzeugt und elektronisch verfügbar sind. Die Kuration, also das Aufarbeiten der Daten, um diese vergleichbar zu machen, ist manuell durchzuführen, zeitintensiv, fehlerbehaftet und damit teuer. Eine logische Konsequenz den menschlich bedingten Dokumentationsmangel zu vermeiden ist daher die Laborautomatisierung, in der man Maschinen bzw. Roboter mit solchen Aufgaben betreut.

Forschungsaufgaben und Experimente haben aber eine sehr große Variabilität in Ihren Arbeitsabläufen, in der Verwendung von Gerätschaften und der Prozessparameter. Eine Laborautomation z.B. durch Robotertechnik ist daher oft zu unflexibel und damit nicht wirtschaftlich. Weiterhin verhindert die Unvorhersehbarkeit des Experimentablaufs die umfängliche Umsetzung einer Laborautomation. Bediener derartiger Roboter sind überwiegend mit der Umsetzung und Anpassung der Experimente auf Roboteranlagen beschäftigt und eingeschränkt auf die Gegebenheiten der Anlagentechnik, was zudem nicht zweckdienlich und ökonomisch ist. Dem meist notwendigen, adaptiven Vorgehen im einzelnen Experiment ist damit nicht gedient, auch leidet darunter der kreative Prozess, der oft erst die Chancen auf den eigentlichen Wissensgewinn ermöglicht.

Weiterhin ändert sich in der Forschung die Betrachtung auf experimentelle Daten je nach Forschungsfragestellung, während die Ausführung der Dokumentation der Experimente häufig von der aktuellen Fragestellung bestimmt wird.

Daher besteht ein großer Bedarf Experimente flexibel durch Menschen durchführen zu lassen und gleichzeitig strukturiert zu dokumentieren, ohne dabei die Forschungsfragestellung ausschließlich im Blick zu haben, sondern vielmehr die experimentellen Informationen für zukünftige Fragestellung exakt aufzuzeichnen und mit Datenauswerteverfahren auch für zukünftige Fragestellungen nutzbar machen zu können.

Weiterhin ist es wirtschaftlich, aufgrund der Grundinvestments, des Unterhalts und der notwendigen Wartung wichtiger Laborressourcen diese gemeinsam von mehreren Experimentatoren nutzen zu können. Zusätzlich sind Forschungskosten oft dominiert von Personalkosten. Die Effizienz des einzelnen Experimentators wird zunehmend reduziert, wenn Laborressourcen nicht geteilt werden können und dadurch die praktisch verfügbare Experimentatorzeit durch vielzählige Wartungsaufgaben verkürzt wird. Zudem hat es eine hohe wirtschaftliche Bedeutung, Fachpersonal so effizient wie möglich zu nutzen.

Aufgabe der vorliegenden Erfindung ist es daher, ein Verfahren bereitzustellen, das es Experimentatoren erlaubt mit geringem Aufwand effizient umfassende Experimentdokumentationen zu erzeugen, ohne die notwendige Variabilität der Versuchsdurchführung einzuschränken und zugleich eine neutrale und objektive, also eine von der Forschungsfragestellung unabhängige, umfassende Dokumentation zu ermöglichen. Eine zusätzliche parallele, undokumentierte Kommunikation zwischen Experimentplaner und Experimentator sowie auch weiteren Dritten soll unnötig gemacht werden. Zudem ist es ein Ziel der vorliegenden Erfindung, eine wirtschaftliche, gemeinschaftliche Nutzung von Laborressourcen zwischen mehreren Experimentatoren zu ermöglichen.

Weiterhin soll das erfindungsgemäße Verfahren vergleichbare Dokumentationen erlauben, die zudem zeitnah elektronisch zur Verfügung stehen. Zudem sollen bereits während der Versuchsdurchführung weitere Informationen und Anweisungen dem Experimentator bereitgestellt werden, die aus früheren Versuchsdurchführungen abgeleitet sind und dem Experimentator ansonsten während der Versuchsdurchführung noch nicht offensichtlich erscheinen. Schließlich soll das erfindungsgemäße Verfahren erlauben, die Daten direkt zur maschinellen Auswertung mit modernen Techniken der Datenwissenschaften bereitzustellen, ohne eine aufwändige Datenkurierung anwenden zu müssen, so dass moderne digitale Verfahren des Machine Learnings (ML) und künstlicher Intelligenz (KI) angewendet werden können.

Diese Aufgabe wird gelöst durch ein computerimplementiertes Verfahren zur elektronischen Aufzeichnung von naturwissenschaftlichen Experimenten in einem elektronischen Laborjournal mit den Merkmalen des Anspruchs 1. Demnach umfasst das erfindungsgemäß Verfahren die folgenden Schritte:
- Bereitstellung wenigstens einer Chemikalie sowie wenigstens eines Laborobjektes und/oder wenigstens eines Labormessgerätes zur Durchführung eines naturwissenschaftlichen Experimentes,
- Registrierung der wenigstens einen Chemikalie sowie des wenigstens einen Laborobjektes und/oder des wenigstens einen Labormessgerätes zumindest für die Zeit ihrer/seiner Verwendung in dem naturwissenschaftlichen Experiment und Verknüpfung mit einer Laborjournal-Experimentkennung jeweils in einem Computersystem,
- Übermittlung wenigstens eines Ereignisses und/oder wenigstens einer Beobachtung zu der wenigstens einen Chemikalie und/oder zu dem wenigstens einen Laborobjekt und/oder wenigstens eines Messergebnisses des wenigstens einen Labormessgerätes an das Computersystem jeweils zusammen mit einem Zeitstempel unter Anwendung der Verknüpfung mit der Laborjournal-Experimentkennung und optional weiterer Verknüpfungsregeln, wobei das Computersystem wenigstens eine Daten-Reihe erzeugt und in einem elektronischen Laborjournal abspeichert.

Der Erfindung liegt die Erkenntnis zugrunde, dass die Durchführung von Experimenten durch eine zeitliche Sequenz von dokumentierten Schritten umfänglich und neutral beschrieben werden kann, wobei der Experimentator die Schritte aufgrund seiner Erfahrung selbständig durchführt, ohne in der Durchführung selbst beschränkt zu sein bzw. ohne eine vollumfängliche, detaillierte Vorplanung der Details des Experimentaufbaus zu benötigen. Eine solche Protokollierung erlaubt es auch dann neutral und strukturiert zu dokumentieren, wenn der Experimentator trotz einer gewissenhaften Planung von selbiger abweicht bzw. in Reaktion auf den Versuchsverlauf abweichen muss.

Erfindungsgemäß wird zur Durchführung und Aufzeichnung eines naturwissenschaftlichen Experiments wenigstens eine Chemikalie sowie wenigstens ein Laborobjekt und/oder wenigstens ein Labormessgerät bereitgestellt. Die erfindungsgemäße Bedeutung der hier verwendetene Ausdrücke "Chemikalie", "Laborobjekt" und "Labormessgerät" wird weiter unten näher erläutert und mit Beispielen unterlegt. Sodann werden die wenigstens eine Chemikalie sowie das wenigstens eine Laborobjekt und/oder das wenigstens eine Labormessgerät zumindest für die Zeit ihrer/seiner Verwendung in dem naturwissenschaftlichen Experiment in einem Computersystem registriert und mit einer Laborjournal-Experimentkennung in dem Computersystem verknüpft. Erfindungsgemäß erfolgt sodann die Übermittlung wenigstens eines Ereignisses und/oder wenigstens einer Beobachtung zu der wenigstens einen Chemikalie und/oder zu dem wenigstens einen Laborobjekt und/oder wenigstens eines Messergebnisses des wenigstens einen Labormessgerätes an das Computersystem jeweils zusammen mit einem Zeitstempel. Dies erfolgt unter Anwendung der Verknüpfung mit der Laborjournal-Experimentkennung und optional weiterer Verknüpfungsregeln, wobei das Computersystem wenigstens eine Daten-Reihe erzeugt und in einem elektronischen Laborjournal abspeichert.

Dabei ergibt die Gesamtheit aller gespeicherten Daten-Reihen enthaltend die Laborjournal-Experimentkennung des naturwissenschaftlichen Experiments bevorzugt dessen elektronische Laborjournaldokumentation. Diese kann nicht nur vom Versuchsplaner und -durchführer, sondern auch von jedem Dritten nachvollzogen werden.

Somit ermöglicht das erfindungsgemäße computerimplementierte Verfahren dem Experimentator ein Höchstmaß an Flexibilität bei der Durchführung seines Experiments, ohne dabei mit aufwändiger Protokollierung belastet zu werden.

Erfindungsgemäß werden die wenigstens eine Chemikalie sowie das wenigstens eine Laborobjekt und/oder das wenigstens eine Labormessgerät zumindest für die Zeit ihrer/seiner Verwendung in dem naturwissenschaftlichen Experiment in einem Computersystem registriert und mit einer Laborjournal-Experimentkennung in dem Computersystem verknüpft. Verknüpfungen mit einer Laborjournal-Experimentkennung und den Kennungen der Laborobjekte, Chemikalien und Labormessgeräte werden bevorzugt in dem Computersystem zwischengespeichert. Unter "Verknüpfung" wird erfindungsgemäß verstanden, dass die genannten Objekte (Chemikalien sowie Laborobjekte und/oder Labormessgeräte) temporär Teil des Laborversuchs mit der ausgewählten Laborjournal-Experimentkennung sind.

Erfindungsgemäß wird ferner wenigstens ein Ereignis und/oder wenigstens eine Beobachtung zu der wenigstens einen Chemikalie und/oder zu dem wenigstens einen Laborobjekt und/oder wenigstens ein Messergebnis des wenigstens einen Labormessgerätes an das Computersystem jeweils zusammen mit einem Zeitstempel unter Anwendung der Verknüpfung mit der Laborjournal-Experimentkennung und optional weiteren Verknüpfungsregeln übermittelt. Die Laborjournal-Experimentkennung wird durch die Anwendung der Verknüpfung im Computersystem ermittelt und zusammen mit einem Ereignis, einer Beobachtung und/oder einem Messergebnis als Daten-Reihe an das Laborjournal gesendet und dort abgespeichert. Die Komponenten der Daten-Reihe für das Laborjournal enthalten demnach a) einen Zeitstempel, b) die Laborjournal-Experimentkennung und c) wenigstens ein Ereignis, wenigstens eine Beobachtung und/oder wenigstens ein Messergebnis zusammen mit d) der Kennung des zugehörigen im Computersystem registrierten Laborobjekts bzw. der Chemikalie bzw. des Labormessgerätes.

Nach der oben beschriebenen erstmaligen Registrierung von Labormessgeräten können diese erfindungsgemäß Messdaten an das Computersystem übermitteln, z.B. in einem zeitlich diskreten, sich wiederholenden Messvorgang oder auch auf Anforderung des Experimentators. Zu den Laborobjekten oder verwendeten Chemikalien können Ereignisse oder Beobachtungen an das Computersystem übermittelt werden. Aus jeder dieser einzelnen auf Labormessgeräte zurückgehenden Übermittlungen an das Computersystem kann dieses somit durch die Verknüpfung mit der Laborjournal-Experimentkennung und wahlweise weiteren Verknüpfungsregeln eine erweiterte Daten-Reihe, beispielsweise mit den Komponenten a) aktueller Zeitstempel, b) Laborjournal-Experimentkennung, c) Ereignis/Beobachtung erweitert um c') einen Messwert und d) der Kennung des registrierten Labormessgeräts erzeugen. Das Computersystem legt diese Daten-Reihe dann im elektronischen Laborjournal ab. In einer bevorzugten Ausführungsform des erfindungsgemäßen Verfahrens wird eine zeitnahe Darstellung der Daten-Reihe dem Experimentator über ein Display/Dashboard bereitgestellt.

Gemäß einer bevorzugten Ausführungsform erfolgt die Registrierung der wenigstens einen Chemikalie, des wenigstens einen Laborobjektes und/oder des wenigstens einen Labormessgerätes und die Verknüpfung mit einer Laborjournal-Experimentkennung mittels einer elektronischen Identifikatoreinheit. Mit anderen Worten: es werden also mit Hilfe einer elektronischen Identifikatoreinheit die im Labor zur Durchführung des naturwissenschaftlichen Experiments bereitgestellte wenigstens eine Chemikalie, das wenigstens eine Laborobjekt und/oder das wenigstens eine Labormessgerät in einem Computersystem anhand einer elektronisch lesbaren Kennzeichnung identifiziert und somit einem einzelnen naturwissenschaftlichen Experiment für dessen Dauer zugeordnet.

Elektronische Identifikatoreinheiten sind Geräte, die dem Experimentator eine schnelle Identifikation von Labormessgeräten, Chemikalien, Laborobjekten, Ereignissen und Beobachtungen erlaubt.

Nach einer weiteren vorteilhaften Ausgestaltung der Erfindung erfolgt auch die Übermittlung des wenigstens einen Ereignisses und/oder der wenigstens einen Beobachtung zu der wenigstens einen Chemikalie und /oder zu dem wenigstens einen Laborobjekt an das Computersystem mittels einer elektronischen Identifikatoreinheit. So können beispielsweise eine mögliche Beobachtungen im Rahmen eines Experiments dem Experimentator bereits als scannbarer Barcode zur Verfügung stehen (z.B. "Lösung beginnt zu sieden" oder "Farbumschlag"), die dann nur noch eingescannt und an das Computersystem übermittelt werden müssen. Auch eine Spracheingabe kann vorgesehen sein.

Nach einer weiteren vorteilhaften Ausgestaltung der Erfindung ist die elektronische Identifikatoreinheit als optische oder akustische Leseeinheit, als Funk-Leseeinheit oder als Messeinheit zur Orstbestimmung ausgebildet.

Optische Identifikatoreinheiten umfassen z.B. optische Barcodeleser, insbesondere Laserscanner, zur Erkennung von Strichcodes (1D-Barcode) und 2D-Barcodes (QR-Code), aber auch einfachere Techniken wie Lichtschranken, Annäherungssensoren, Taster und Schalter. Auch mehrere verschiedene Identifikatoreinheiten können kombiniert werden. Die Laborobjekte, Chemikalien und Labormessgeräte tragen daher bevorzugt eine kompatible lesbare Identifizierung (auch "Tag" oder "Barcode"/"QR-Code" genannt) und können mit der elektronischen Identifikatoreinheit durch Annäherung und Ausrichtung der elektronischen Identifikatoreinheit authentifiziert werden. Alternativ kann die Identifikatoreinheit als Funk-Leseeinheit, bevorzugt als RFID-Leseeinheit, insbesondere als auf dem NFC-Standard basierende RFID-Leseeiheit, ausgebildet sein.

Weiterhin kann die elektronische Identifikatoreinheit als Messeinheit zur Orstbestimmung ausgebildet sein. Eingesetzt werden bevorzugt Techniken zur Ortsbestimmung mittels Satellitentechnik wie z.B. das amerikanische NAVSTAR-GPS (global positioning system), das europäische Galileo (GNSS), das russische GLONASS, das chinesische Beidou, das Starlink und die Innenraumortsbestimmung (IPS), die mittels Radiofrequenzverfahren wie z.B. WIFI, Bluetooth, optische, akustische (Ultraschall) oder andere Methoden den Ort eines markierten Laborobjektes oder eines Labormessgerätes bestimmen können. Die Markierung erfolgt dabei häufig über eine Identifikationstag (Beacon). Der Experimentator kann hiermit die Identifikation und die bestimmungsgemäße Verwendung eines Laborobjektes oder eines Labormessgerätes durch die Positionierung an einem Ort festlegen. Diese Techniken lassen sich insbesondere für track & trace Anwendungen von Probentransporten zwischen zusammen arbeitenden Laboren verwenden.

Ferner kann die elektronische Identifikatoreinheit als optisches Lesegerät, insbesondere als Kamera, ausgebildet sein. Optische Kameras erlauben durch die Vielfältigkeit Ihrer Funktion sowohl die Identifikation als auch eine Verwendung als z.B. optischer Sensor. So kann eine Kamera gleichzeitig optischer Scanner sein, um ein Laborobjekt oder Labormessgerät zu identifizieren und seinen Ort als bestimmungsgemäße Verwendung festzulegen. Beispiele sind hier die Identifikation eines Temperatursensors durch seinen QR-Code und die Bestimmung des Ortes des Temperatursensors. Algorithmen, die mittels maschinellem Lernen trainiert wurden, können auch Kameras zu Identifikationseinheiten machen, die noch speziellere bestimmungsgemäße Verwendungen erlauben und die weitere Identifikationsschritte überflüssig machen. So kann eine Kamera mehrere Laborobjekte gleichzeitig erkennen und darüber die bestimmungsgemäße Verwendung festlegen. Beispielsweise kann ein Gefäß, dass auf eine Waage gelegt wird, automatische gewogen werden (als Taraeinstellung der Waage) und dann mit einer Chemikalie gefüllt wird. Die Kamera erkennt die Identität des Gefäßes, der Waage und der Chemikalie am Gebindeetikett und kann zudem der Waage zugleich das Ende der Befüllung anzeigen, die dann das Taragewicht und das Einwaage-Gewicht der Chemikalie elektronisch mitteilt.

Nach einer weiteren vorteilhaften Ausgestaltung der Erfindung kann die Identifikatoreinheit als System verteilter Sensoren ausgebildet sein. Diese können beispielsweise als im Labor angeordnete optische Sensoren (Lichtschranke) oder Bewegungssensoren ausgebildet sein und eine automatische Registrierung der Chemikalien, Laborobjekte oder Labormessgeräte ermöglichen.

Chemikalien im Sinne der vorliegenden Erfindung umfassen Rohstoffe, die in organischen und anorganischen Synthesen verwendet werden wie z.B. Feinchemikalien, Reagenzien, technische Rohstoffe, Lösemittel, Reaktionsmischungen, Syntheseprodukte ("Produkt"), Zwischenstufen einer Synthese oder einer Präparationen, Probe einer Probennahme, Lösungen z.B. in definierten Lösemitteln und Gehalten ("10%ige wässrige Lösung", "80% Lösung in Butylacetat").

In bestimmten Industrien und Anwendungsgebieten gibt es weiterhin spezielle Funktionsbeschreibungen (funktionale Bezeichnungen) für Rohstoffe, die ebenfalls hier als Chemikalien bezeichnet werden:
Für Lacke sind dies z.B. ein oder mehrere Komponenten ausgewählt aus Bindemittel, wahlweise einem oder mehreren Vernetzern, Füllern, Pigmenten und Farbstoffe, Farbpasten, Verlaufshilfsmittel, Entschäumer, Entgaser, Adhäsionsverbesserer, Stabilisatoren wie Antioxidantien und Lichtschutzmittel, Dispergier- und/oder Rheologieadditive, Oberflächenmodifikatoren wie Mattierungsmittel oder Texturierungsmittel, Lösemittel und/oder Wasser, Cosolventien, (Farb-)-Masterbatches, Initiatoren und/oder Katalysatoren, Corrosionschutzmittel.

Für Klebstoffe und Dichtmassen sind dies z.B. Bindemittel (z.B: semikristalline Polymere für Schmelzklebstoffe, Dispersionen für wässrige Systeme), Lösemittel, Tackifier, Vernetzer, Katalysatoren, Füller, Weichmacher, Stabilisatoren, Thixotropiermittel, Beschleuniger sowie Farb- oder Füllstoffe.

Für Gießharze sind dies z.B. Bindemittel und Vernetzer, Flammschutzmittel, Katalysatoren, Weichmacher, Stabilisatoren, Beschleuniger sowie Farb- oder Füllstoffe und Additive.

Für Schäume sind dies z.B. Polyol und Isocyanat, thermoplastische Polymere (z.B: Polyethylen, Polypropylen, Polystyrol, Polycarbonat, thermoplastischen Polyurethane), Treibmittel, Wasser, Gase oder Luft, Katalysatoren, Stabilisatoren, Beschleuniger, Füllstoffe, Flammschutzmittel und Additive.

Für Kosmetikprodukte sind dies z.B. Bindemittel, Polymere, Duftstoffe, Weichmacher, Füllstoffe, Pigmente, Farbstoffe, Mattierungsmittel, Wachse, Fette, Öle, waschaktive Substanzen, Stabilisatoren, Nährstoffe, Lichtschutzmittel, Emulgatoren, Konservierungsmittel, Pflanzenstoffe, Aktivstoffe.

Für Lebensmittel sind dies z.B. die Rohstoffe und Mischungen von Kohlenhydraten, Eiweiße/Proteine, Fette, Vitamine, Mineralien, Ballaststoffe, Wasser, Salze, Emulgatoren, Rohstoffe pflanzlichen oder tierischen Ursprungs, Geschmacksstoffe, Geruchsstoffe, Farbstoffe, wobei auch Rohstoffe Lösungen oder Gemische dieser Rohstoffe selbst darstellen können.

Für Thermoplaste und Thermosets sind dies z.B. Weichmacher, Stabilisatoren, Lichtschutzmittel, Flammschutzmittel, Aktivatoren, Vernetzer, Farbstoffe, Füllstoffe, Entformungsmittel;
Für Druckfarben sind dies z.B. Bindemittel, Lösemittel und Wasser, Farbstoffe, Dispergieradditive, Farbpasten, Photoinitiatoren, Benetzungsmittel, Additive, Tribologiemittel, Verdicker, Ammoniak, Katalysatoren und Trocknungsmittel, Granulate, Filamente, Tinten.

Für Pharmazeutika und Pflanzenschutzmittel sind dies Wirkstoffe, Lösemittel, Emulgatoren, Aktivatoren, Dispergiermittel, Farbstoffe, Frostschutzmittel, Haftmittel, Verdicker, Konservierungsmittel, Fließmittel, Netzmittel, Parfüme, Treibgas, Repellents, Stabilisatoren, Trägerstoff, Schmiermittel, Schaumverminderer, Puffer, Bindemittel, Füllstoffe.

Weitere Chemikalien im Sinne dieser Erfindung sind Prüfkörper, wie z.B. Prüfstäbe für Zugversuche, spritzgegossene Musterplatten, Schichtaufbauten und beschichtete Substrate, Proben; Mustervorbereitungen wie KBr Presslinge für die IR-Spektroskopie, Lösungen in deuterierten Lösemitteln in der NMR-Spektoskopie, gefüllte Tiegel für DSC und TGA Analysen etc; Rohstoffe und Produkte als auch Formulierungen in Standardgefäßen, wie z.B. Tablettenröhrchen und/oder Küvetten, gestanzte Körper aus Schichtaufbauten und/oder aus Filmen mit definierte Schichtdicke.

Laborobjekte, hier auch physische Laborobjekte genannt, umfassen Gebinde, Reaktoren, Dosiergefäße, wobei hier erfindungsgemäß in der Natur dieser Objekte weiter unterschieden wird, die die Größe oder das Fassungsvermögen, die Materialbeschaffenheit, die Form aber auch gebräuchliche fachspezifische Namen umfassen können z.B.: 250 ml 3-Halskolben, 500 ml Polyethylenflasche, 2 Liter Planschlifftopf, 1 Liter Glasflasche, 250 ml Erlenmeyerkolben, 200 ml Glastroptrichter mit Gasausgleich und drgl.

Weitere Laborobjekte sind Mischaggregate, so z.B. Rührwerke, Dispergatoren, Extruder, Mehrkomponentenmischaggregate wie z.B. Statikmischer, Mischkammern und Reaktivextruder, Schäumanlagen, Pelletieraggregate, Mischer, Rührer, Ultraturrax, Ultraschallbäder, Sprühpistolen und drgl..
Weitere Laborobjekte sind Temperiergeräte, wie z.B. Heizbäder, Heizpilze, Temperiermäntel, Peltierelemente, Temperierbäder, Umlaufpumpen, Infrarotstrahler, Kühlschränke, Wärmeschränke, Laboröfen, Trockner, Heizstrahler;
Weitere Laborobjekte sind Apparaturen zur Synthese und Aufreinigung, wie Reaktionskolben, Planschifftöpfe, Druckapparaturen, Stahlreaktoren, Destillationskolonnen, Rückflußkühler, Destillationsbrücken, Destillationsspinnen, Auffanggefäße, Dünnschichtfilmverdampfer, Chromatographiesäulen und -platten, Kristallisationsgefäße, Impfkristalle, Dosiergefäße, Dosierpumpen und Schläuche, Tropftrichter, Erlenmeyerkolben, Auffanggefäße, Trocknungsmittel, Schütteltrichter, Rotationsverdampfer;
Wenn Laborobjekte zugleich auch Messsensoren enthalten, werden diese Messsensoren im Sinne dieser Erfindung als Labormessgeräte betrachtet.

Erfindungsgemäß kann mit einem Laborobjekt auch ein Rührplatz, ein Verarbeitungsort, eine Apparatur, ein Labor, ein Forschungsbereich bezeichnet sein.

Labormessgeräte umfassen Waagen, Temperatursensoren, Viskositätsmesser, Dichtemesser, Schaumdichtemesser, Zellstrukturanalysatoren, Trübungsmessgeräte, Manometer, Dosierwaagen, Titratoren, Pipetten, Büretten, sowie allgemein Messgeräte für Messparameter, wie z.B. mechanische Größen (wie z.B. Kräfte, Elastizitätsmodul, Reißdehnung, und dgl.), optische Größen (z.B. Brechungsindex, optische Retardation u. dgl.), Schaumstrukturen (wie z.B: Schaumdichte, Zellform, Zellwandstärken, Zelldurchmesser).

Weitere Labormessgeräte umfassen Geräte zur stofflichen Mengenbestimmung und Charakterisierung bzw. Fingerprintanalyse, wie z.B. Spektroskope (Infrarot (IR), Raman, UV/VIS, Nahinfrarot, Kernresonanz (NMR), Flammenspektroskopie, Emissionsspektroskopie, Brechnungsindex, ...), Spektrometer (Massenspektrometrie mit verschiedenen Ionisationsverfahren wie Elektronenstossionisation, chemischer Ionisation, Elektrosprayionisation, Matrix assistierter Laserdesorptionsionisation), Röntgendiffraktometrie, Trennverfahren mit Sensoren wie z.B. Flüssigchromatographie, Gaschromatographie, Elektrophorese, Immunoassays, biochemische Methoden wie PCR, Titratoren mit z.B. Farbumschlagsreaktionen, Voltametrie, Leitfähigkeitssensoren, Säure-Base-Titrationen und drgl.

Weitere Labormessgeräte umfassen Geräte zur Bestimmung von stofflichen Kenngrößen, wie z.B. Wassergehalt, Elementgehalte, Festkörpergehalt, Säurezahl, OH-Zahl, Isocyanatgehalt, Epoxygehalt.

Weitere Labormessgeräte umfassen Geräte zur Bestimmung von physikalischen oder anwendungstechnischen Eigenschaften, wie z.B. Viskosität, Rheologie, thermische Eigenschafte wie Glasübergang oder Schmelzpunkt/bereich, thermischer Massenverlust, Schmelzpunkt, Schaumdichte, Flammpunkt, Feuerbeständigkeiten, Härten, Schlagfestigkeiten, Weiterreißfestigkeit, Chemikalienbeständigkeiten, Ermüdung, Teilchengrößen und Teilchengrößenverteilung, Trockungszeit, Kratzbeständigkeiten, Adhäsionskraft, Festkörpergehalt, Haptik, Topfzeit, Gelzeit, Brennwert, Dichte, Sedimentations- und Aufcremebestimmung, Schaumstabilität, Oberflächenrauhigkeit, Glanzgrad, Trübung, Abzugskraft, sowie Ergebnisse der Materialprüfung aus Dynamisch-Mechanische Analyse, Thermomechanische Analyse, Dielektrizitätsspektroskopie, Zugprüfung, Schmelzindex, Farbmetrik, Bewitterungstests, Schichtdicken, UV-, VIS-, nIR-Messung.

Weitere Labormessgeräte sind Sensoren, die eine Handlung des Experimentators aufzeichnen. So können dies Schalter, Lichtschranken, Mikrophone, Kameras, Näherungssensoren, Barcode, RFID-Lesegeräte und dergleichen sein, die weitere Aktionen eines Experimentators - sowohl mit als auch ohne sein aktives Zutun - in einem Versuchsverlauf dokumentieren. Der Messwert derartiger Sensoren kann binär sein (an/aus), umfangreiche Meßdaten enthalten (Audio und Videosignale) oder auch kodierte Daten enthalten. Auch ist er möglich die Auswertung von Daten durch das Labormessgerät selbst zu ermöglichen und nur einen Datenwert zu übertragen.

Ein Ereignis in einem naturwissenschaftlichen Experiment wird als Nachricht, z.B. durch eine elektronische Identifikatoreinheit, an das Computersystem übermittelt. Vorzugsweise werden Ereignisse vordefiniert und der Experimentator kann aus den vordefinierten Ereignissen z.B. mit einen Identifikator eines auswählen. Über eine zwischengespeicherte Verknüpfung mit der Laborjournal-Experimentkennung, bevorzugt wiederum unter Verwendung einer elektronischen Identifikatoreinheit, erzeugt das Computersystem Daten-Reihen, die a) den Zeitpunkt in Form eines Zeitstempels b) die Laborjournal-Experimentkennung, c) die Kennung des Ereignisses und d) Kennung der Chemikalie/des Laborobjektes/des Labormessgerätes und optional eine weitere Unterbeschreibung der Art des Ereignisses umfasst (wie z.B. eine Beobachtung einer Farbe oder Trübung).

Die Dokumentation von Ereignissen ist besonders hilfreich, um eine Analyse der Daten mittels maschineller Auswertung mit modernen Datenanalysemethoden zu erleichtern. Insbesondere unterstützen Ereignisse zudem die visuelle Analyse der Daten.

Im folgenden werden Beispiele von Ereignissen im Sinne der Erfindung genannt: So sind Beispiele für Ereignisse in einer Synthese etwa der Beginn bzw. Ende der Beladung des Reaktors, Start der Temperierung des Reaktors, Beginn und Ende des Eindosierens eines Rohstoffs, der Beginn und das Ende der Reaktionszeit, Beginn und Ende der Nachrührzeit, Zeitpunkt einer Probennahme, Beginn der Entleerung eines Reaktors oder das Ende der Synthese. Insbesondere dienen Ereignisse einer zeitlichen Unterteilung eines Experiments und erfolgen zu bestimmten Zeitpunkten, die es erlauben die Vielzahl von Vorgangsphasen zu strukturieren. So ist es auch möglich, das Ereignis "Prozessphase" zu definieren und jeweils eine Unterbeschreibung mit anzugeben, z.B. "Beginn der Reaktorbeladung", "Beginn der Aufheizung", "Beginn der Reaktion", "Beginn der Abkühlungsphase", "Beginn der Entladung" und "Ende der Entladung".

Ereignisse eignen sich zur Analyse von Formulierungsversuchen, also bei Prozessschritten, die ein Vermengen bzw. Vermischen von Rohstoffen beschreiben. Typische Anwendungsgebiete der Formulierungsindustrien sind Lacke, Klebstoffe, Kosmetika, Lebensmittel, Druckfarben, Pasten, Dichtstoffe, Zement- und Betonindustrie, Bauwerkstoffe, Schäume, Vergussmassen, Pflanzenschutzmittel, Therapeutika, Gießharze, Schlichten, Textilbeschichtungen, Gummimischungen, Komposite und Thermoplaste.

Weiterhin kann ein Ereignis den Zeitpunkt der Einstellung oder Änderung der Einstellung eines Laborobjektes dokumentieren, z. B. eine neue Einstellung einer Temperatur eines Bades, eines Rührwerks, eines Zieldrucks einer Pumpe o. drgl. Derartige Ereignisse eignen sich insbesondere, um die Funktionsfähigkeit, die Funktionskonstanz, die Trägheit von Einstellungen und Regelsteuerungen zu untersuchen und vergleichbar zu machen.

Ereignisse können auch Meldungen von Sensoren oder Messgeräten sein, so zum Beispiel das Über- oder Unterschreiten eines Temperaturbereiches, eine Überdruckwarnung, Überschreiten eines Strombedarfs eines Rührwerks, eine Überfüllmeldung eines Reaktors.

Ereignisse können auch eine Beschreibung/Annotation des Experimentators zu einem Zeitpunkt oder einer zeitlichen Phase eines Experiments sein. So können damit eine Lot- oder Batchnummer, eine Qualitätsmanagementnummer, ein Name eines typischen Verarbeitungsschrittes, eine Numerierung, eine Messbedingung sein. Weiterhin kann ein Ereignis auch eine chemische Beschreibung umfassen, die das Syntheseverfahren und/oder den Chemismus beschreiben wie z.B. Veresterung, Veretherung, Urethanisierung, Dimerisierung, Trimerisierung, Oligomerisierung, Metathese, Ringöffnung, Amidierung, Nitrierung, Heterogene Katalyse, Halogenierung, Sulfonierung, Grignard, Acetylierung, Alkylierung, Acylierung, Reduktion, Hydrierung, Aminierung, elektrophile Substitution, nukleophile Substitution, Eliminierung, Kondensation.

Beobachtungen sind vordefinierte qualitative Beschreibungen von Experimenten und betreffen z. B. das Maß einer Trübung ("keine Trübung", leichte Trübung", ...), das Maß eine Phasenseparation ("leichter Niederschlag", "viel Niederschlag"), eine Phasentrennung, Färbung, oder Gelierung. Beobachtungen sind auch standardisierte visuelle Beurteilungen basierend auf industrieüblichen Test- und Prüfverfahren: So ist es in z.B. in der Lackindustrie üblich, Ergebnisse bestimmter Testverfahren mit Kennziffern und/oder Buchstaben zu bewerten (z.B. Gitterschnittprüfungen nach DIN EN ISO 2409 in _{"}0", _{"}1", _{"}2", _{"}3", _{"}4", _{"}5"; Bleistifthärte nach DIN EN ISO 15184, ASTM D 3363 in 6B, 5B, 4B, 3B, 2B, B, HB, 1H, 2H, 3H, 4H, 5H, 6H usw.).

In dem erfindungsgemäßen Verfahren wird der Zeitpunkt einer Messung, eines Ereignisses oder einer Beobachtung bestimmt, so dass das Messergebnis, das Ereignis oder die Beobachtung mit einem Zeitstempel an das Computersystem übermittelt werden kann. Dabei kann die Zeiterfassung im Labormessgerät selbst und/oder in einer Identifikatoreinheit erfolgen oder auch durch das Computersystem im Verlauf der Datenverarbeitung erfolgen. Mit einem Zeitstempel im Sinne dieser Erfindung ist also die protokollierte Zeiterfassung in einem Computersystem gemeint.

Nach einer weiteren vorteilhaften Ausgestaltung der Erfindung kann das Computersystem weitere Verknüpfungsregeln anwenden, insbesondere derart, dass die wenigstens eine Chemikalie, das wenigstens eine Laborobjekt und/oder das wenigstens eine Labormessgerät einander zugeordnet werden. Dabei ist bevorzugt vorgesehen, dass die Zuordnung auf einer bestimmungsgemäßen Verwendung des wenigstens einen Laborobjekts und/oder des wenigstens einen Labormessgeräts, einer zeitlichen Reihenfolge der Registrierung der wenigstens einen Chemikalie, des wenigstens einen Laborobjekts und/oder des wenigstens einen Labormessgeräts, der relativen räumlichen Position der wenigstens einen Chemikalie, des wenigstens einen Laborobjekts und/oder des wenigstens einen Labormessgeräts zueinander, oder einer beliebigen Kombination der vorstehenden basiert.

Derartige weitere Verknüpfungsregeln nutzen somit die bestimmungsgemäße Verwendung von Laborobjekten, Labormessgeräten, Ereignissen, Beobachtungen und deren Anwendung auf andere Laborobjekte und Chemikalien. Beispielhaft sei die weitere Verknüpfungsregel eines Labormessgeräts zur Gewichtsbestimmung (Waage) genannt, die die Anwendung auf bestimmte Chemikalien und/oder Gebinde beschränkt (Waage wiegt zunächst (ausschließlich) Gefäß X und sodann (ausschließlich) Gefäß X mit Chemikalie Y darin). Bestimmungsgemäße Verwendungen können auch Funktions- und Messmethoden von Labormessgeräten definieren, als auch den Ort eines Labormessgerät festlegen.

Weitere Verknüpfungsregeln, die die zeitliche Reihenfolge berücksichtigen, sind insbesondere zweckmäßig, um übliche Handlungsabläufe abzubilden, wie z.B. die Einwaage von Chemikalien. Die weitere Verknüpfungsregel wird vorab festgelegt, z.B. kann diese Verknüpfungsregel die Registrierung einer Chemikalie oder eines Laborobjekts (z.B. Becherglas oder Kolben), und danach die Registrierung einer Waage betreffen, um festzulegen, welche Chemikalie bzw. welches Laborobjekt diese Waage konkret wiegt. Die mit der Verknüpfungsregel durch das Computersystem erzeugten Daten-Reihe enthält nun die Komponenten a) aktueller Zeitstempel, b) Laborjournal-Experimentkennung, c) Messwert, d) Kennung des registrierten Labormessgeräts (hier also die Waage) und e) Kennung der registrierten Chemikalie und/oder des registrierten Laborobjekts. Eine zeitliche weitere Verknüpfungsregel kann vorsehen, dass zeitlich unmittelbar nacheinander registrierte Laborobjekte/Chemikalien/Labormessgeräte miteinander verknüpft werden. Alternativ kann ein/e zuletzt registrierte/s Laborobjekt/Chemikalie/Labormessgerät mit einem/r bereits registrierten, jedoch noch nicht zugeordneten Laborobjekt/Chemikalie/Labormessgerät verknüpft werden.

Weitere Verknüpfungsregeln, die die bestimmungsgemäße Verwendung berücksichtigen, sind insbesondere zweckmäßig, um die Eineindeutigkeit von Verknüpfungen sicherzustellen. So ist die Anwendung von Labormessgeräten zweckmäßig einschränkbar auf Laborobjekte, die auch technisch sinnvoll durch ein Labormessgerät messbar sind oder die in der Laborpraxis auf solche Laborobjekte angewendet werden, z.B. kann ein Temperaturmesssensor die Temperatur einer bestimmten Chemikalie, die Innentemperatur von Reaktoren oder Gefäßen oder auch die Umgebungstemperatur messen. Die bestimmungsgemäße Verwendung schränkt damit die für einen speziellen Temperatursensor zu verknüpfenden Laborobjekte für das Computersystem ein, die damit gemessen werden sollen oder können.

Zudem ist es zweckmäßig Verknüpfungsregeln festzulegen, die die zeitliche Verwendung und die bestimmungsgemäße Verwendung kombinieren. So kann in einem Einwaageprozess festgelegt werden, dass zuerst eine Chemikalie mit einer elektronischen Identifikatoreinheit und dann anschließend die Waage mit der elektronischen Identifikatoreinheit registriert wird, danach erfolgt die Übermittlung der Einwaage durch die Waage. Alternativ kann die Verknüpfungsregel auch lauten, dass zunächst eine Waage mit einer elektronischen Identifikatoreinheit registriert wird, anschließend werden alle einzuwiegenden Chemikalien registriert und dann erfolgt die Übermittlung der Einwaagen in genau dieser Reihenfolge der zuvor registrierten Chemikalien. Vorteile derartiger Verknüpfungsregeln sind die Minimierung geforderter Registriervorgänge mit einer elektronischen Identifikatoreinheit.

Es kann zudem vorgesehen sein, dass eine weitere Verknüpfungsregel durch den Experimentator über ein Auswahlmenü in einem Display, Dashboard, oder ein anderes Mensch-Maschine-Interface festgelegt wird.

Die Gesamtheit aller durch das Computersystem im Laborjournal gespeicherten Daten-Reihen mit der Laborjournal-Experimentkennung eines naturwissenschaftlichen Experiments stellen somit bevorzugt die elektronische Dokumentation des naturwissenschaftlichen Experiments im Laborjournal dar. Die Anzahl der Komponenten dieser Datenreihen kann unterschiedlich sein, beinhaltet aber immer zumindest a) einen Zeitstempel, b) eine Laborjournal-Experimentkennung, c) eine Beobachtung oder ein Ereignis und wahlweise weitere Verknüpfungen, d) die Kennzeichung eines registrierten Laborobjekts, einer registrierten Chemikalie und/oder eines registrierten Labormessgeräts.

Die Umsetzung des erfindungsgemäßen Verfahrens in die Praxis erfordert Vorbereitungen zur Registrierung von Laborobjekten, Chemikalien und Labormessgeräten in zumindest einem oder auch in mehreren Laborbereichen, die jeweils aus einem oder auch mehreren Laboren bestehen können, wobei zudem zusätzlich unterstützende Laborbereiche wie Analytik- und Messräume, Chemikalienlager, Reinigungsplätze, Applikationsräume, Logistikbereiche und dergleichen berücksichtigt werden können. Generell gilt, dass Synergien umso größer sind, je umfassender derartige Laborbereiche einbezogen werden. Vorzugsweise ist die Kennzeichnung dieser Laborbereiche derart gestaltet, dass diese, insbesondere durch eine elektronische Identifikatoreinheit, elektronisch lesbar ist.

Im Folgenden sollen die weiteren Verknüpfungsregeln erläutert werden, die bestimmungsgemäße Verwendungen nutzen. Tabelle 1 zeigt ausgewählte Labormessgeräte und deren mögliche bestimmungsgemäße Verwendung auf andere Laborobjekte. Unter einer bestimmungsgemäßen Verwendung eines Messgeräts wird dabei die vom Experimentator bestimmte Anwendung auf ein Laborobjekt oder eine Chemikalie verstanden. Die weitere Verknüpfungsregel kann auch derart gestaltet sein, dass mehrere Laborobjekte einem Labormessgerät, einem Ereignis und/oder einer Beobachtung zugeordnet werden: z.B. kann zusätzlich eine Methodenauswahl oder ein Messparameter einem Labormessgerät zugeordnet sein; einem Ereignis oder einer Beobachtung kann zusätzlich ein Ort und/oder ein Chemismus zugeordnet sein. Die Festlegung einer konkreten Verwendung eines Labormessgeräts auf ein Laborobjekt im konkreten Experiment erfolgt erst in der Experimentdurchführung durch den Experimentator mit der Nutzung des Identifikators und der Labormessgeräte, insbesondere durch die Reihenfolge der Registrierung in dem Computersystem. Die weitere Verknüpfungsregel einer bestimmungsgemäßen Verwendung beschreibt dabei, welche Laborobjekte durch ein Labormessgerät gemessen bzw. verwendet werden dürfen.

**Tabelle 1: Beispielhafte Definition von bestimmungsgemäßen Verwendungen**

| Labormessgerät | Beispielhafte bestimmungsgemäße Verwendung auf Laborobjekte |
|---|---|
| Waage | Rohstoff, Reaktor, Gebinde, Dosiergefäß |
| Rheometer | Rohstoff, Produkt, Probe |
| Zugprüfmessgerät | Zugversuchprobe |
| pH-Meter | Rohstoff, Lösung, Produkt |

Neben weiteren Verknüpfungsregeln, die auf der zeitlichen Reihenfolge basieren, lassen sich auch Verknüpfungsregeln über die örtliche Nähe von physischen Laborobjekten zueinander und/oder die Bewegungsrichtung von Laborobjekten definieren. Hierzu sind insbesondere technische Verfahren relevant, die den Ort und/oder Bewegung von Laborobjekten bestimmen. Beispielsweise kann durch eine weitere Verknüpfungsregel ein Laborobjekt/eine Chemikalie/ein Labormessgerät mit demjenigen anderen verknüpft werden, welches einen minmalen Abstand zum erstgenannten aufweist. Auch kann der Ort des Laborobjekt/der Chemikalie/des Labormessgerät, in absoluten Koordinaten bestimmt durch ein Innenraumpositionierungsverfahren oder ein globales Positionierungsverfahren (GPS,IPS) oder aber in relativen Koordinaten (z.B. ein/e ortsvariable/s Laborobjekt/Chemikalie/ Labormessgerät ist in der Nähe eines/r anderen ortsvariablen Laborobjekts/Chemikalie/ Labormessgeräts - beispielsweise eine Materialprobe, die sich in der Nähe eines tragbaren Labormessgeräts befindet) ein Kriterium für eine weitere Verknüpfungsregel sein.

Weitere erfindungsgemäße Verknüpfungsregeln sind die Verwendung von Kameras, die eine Identifikation durch Bilderkennung (in Form eines Barcodes, QR-Codes, durch eine direkte Objekterkennung mittels Machine Learning) und eine bestimmungsgemäße Verwendung (z.B. durch Ortbestimmung im Blickfeld der Kamera, durch die relative Position zu einem anderen Objekt) erlauben.

Weitere erfindungsgemäße Verknüpfungsregeln basieren auf Spracherkennung, so z.B. kann der Experimentator seine Handlung sprachlich kommentieren und damit sowohl eine Registrierung eines Laborobjektes/einer Chemikalie/eines Labormessgerätes und die Festlegung der bestimmungsgemäßen Verwendung durchführen. Ebenfalls lassen sich auch Ereignisse und Beobachtungen mit Spracherkennung erzeugen und erfindungsgemäß dokumentieren.

Weitere erfindungsgemäße Verknüpfungsregeln erlauben die Registrierung von Laborobjekten/Chemikalien/Labormessgeräten mittels Sensoren und Messverfahren, die auf mehrere ortsnahe Experimente anwendbar sind. So ist es besonders bevorzugt, Klimasensoren, Raumsensoren auf mehrere Experimente anzuwenden, wobei deren bestimmungsgemäße Verwendung der Ort des Sensors ist. So kann z.B. mit dieser Verknüpfungsregel ein Feuchtigkeitssensor in einem Laborraum allen darin durchgeführten Experimenten zugeordnet werden.

Weitere erfindungsgemäße Verknüpfungsregeln nutzen die zeitliche Auswertung von Ortspositionen zur Ermittlung einer Richtung, konkret hier der Bewegung, sowie das Verweilen an einem Ort. Auch geeignet sind Labormessgeräte, die eine Bewegung/Beschleunigung messen können wie z.B. MEMS-Bewegungssensoren.

Im Folgenden werden erfindungsgemäße Beispiele für Verknüpfungsregelen genannt, die durch die Nutzung von Orts-, Bewegungs- und Zeitsensoren ohne weitere Aktion des Experimentators festgelegt und dokumentiert werden.

Mit einer weiteren Verknüpfungsregel des Ortes ist beispielsweise der Messort eines Temperatur- oder Feuchtesensors gemeint.

Ein Beispiel für eine Verknüpfungsregel durch eine örtliche Information zu Laborobjekten sei an folgender Variante erläutert, die eine Viskositätsmessung spezifiziert, die erst durch die Auswahl der Geometrie und Größe einer Spindel hinreichend definiert ist: Durch die örtliche Nähe a) eines Rheometers und b) einer zu dem Rheometer passenden Rheologie-Spindel wird der Messparameter des Rheometers festgelegt und damit die Rheologiemessung präzisiert und die Messwerte erst auswertbar. Die örtliche Nähe kann dabei durch ortsfeste Identifikatoren erfolgen, durch eigene Ortsbestimmung der Laborobjekte, Chemikalien und Labormessgeräte. Die Verknüpfungsregel selbst besteht aus der Berechnung von räumlichen Abständen auf Basis der Ortsinformationen selbst.

Ein weiteres Beispiel für eine weitere Verknüpfungsregel durch eine zeitliche Reihenfolge, insbesondere die zeitliche Nähe der Registrierung, von Laborobjekten sei an einem Rakelauftrag eines Lackes beschrieben: Aus der zeitlich nahen, bevorzugt unmittelbar aufeinander folgenden, Registrierung a) eines Gebindes enthaltend eine Lackformulierung und b) eines Musterpanels einer Substratsorte (z.B. Holzpanel aus Birke oder Holzpanel aus Eiche), die beide bevorzugt von einem, insbesondere von demselben, Identifikator registriert werden, erhält der ebenfalls registrierte Rakelauftrag somit die Verknüpfung der Lackformulierung und der Substratsorte. Letztere werden in der Daten-Reihe als weitere Komponente im Laborjournal abgespeichert.

Beispiel einer Verknüpfungsregel durch eine Bewegungsrichtung ist die Bewegung der Hand eines Experimentators bei einem Rakelversuch, wodurch Geschwindigkeits- und Beschleunigungswerte das Experiment detaillierter dokumentieren. Technisch ist die Erfassung von zeitabhängigen Bewegungen mittels Bewegungssensoren an der Hand oder am Handgelenk möglich, wie sie heute in vielen mobilen Konsumelektroniken (z.B. Mobiltelefonen, Sportarmbänder) weit verbreitet sind und wahlweise personalisiert vom Experimentator getragen werden. In diesem speziellen Fall ist die Zeitreihe der Geschwindigkeits- und Beschleunigungswerte des manuellen Rakelvorganges in der Daten-Reihe eine weitere Komponente, die im Laborjournal abgespeichert wird.

Weiterhin kann nach einer weiteren bevorzugten Ausführungsform durch die einmalige Registrierung eines Labormessgeräts zeitgleich eine Verknüpfung zu einer Messmethode und/oder einem Laborobjekt festgelegt werden. Bevorzugt ist die Verknüpfung zu einer Messmethode des Labormessgerätes. So kann z.B. bei einem Titrator zur Bestimmung eines Isocyanat- oder Epoxygehalts durch einen Barcode zugleich der Titrator und der Messbereich der durchzuführenden Bestimmung zugeordnet werden. Nutzt man denselben Titrator nun für verschiedenen Analyseverfahren bzw. Analyt-einwaagen, ist es nach einer weiteren vorteilhaften Ausgestaltung der Erfindung zudem bevorzugt, verschiedene Barcodes/Tags zur Identifikation zu verwenden, die jeweils den Titrator, das Messverfahren bzw. den Messbereich definieren. Der besondere Vorteil ist hierbei die vereinfachte Nutzung für den Experimentator, der dann nur eine statt mehrere Indentifikationsschritte durchzuführen braucht.

Komponenten der Daten-Reihe können somit vielfältige Datenformate haben, umfassend i) Texte (Strings) z.B. für Kennungen und kategoriale Variablenwerte, ii) reale Zahlen für z.B. Messwerte, iii) Zahlenkolonnen für z.B. eine Serie von Messwerten, iv) Tabellen für z.B. Messwertpaare (z.B. Beschleunigungswerte mit Zeiteinheiten), v) komplexere Datenformate für z.B. Sensoren und Labormessgeräte (Bilddaten, Videodaten, Messgeräte-Rohdaten enthaltend Messgeräteparameter und Messdaten).

Labormessgeräte und Laborobjekte können auch zusätzlich noch eine eigene autonome Aktivität haben, die weitere Daten-Reihen im Computersystem mit jeder Aktivität fortan erzeugen. Autonome Aktivitäten sind meist getriggert durch Zeitintervalle und/oder Messwertänderungen. Tabelle 2 zeigt beispielhaft bestimmungsgemäße Verwendungen auf andere Laborobjekte und ein Beispiel der Aktivität.

**Tabelle 2: Beispielhafte Definition von Laborobjekten/Labormessgeräten mit zusätzlich eigener Aktivität**

| Laborobjekt / Labormessgerät | Bestimmungsgemäße Verwendung | Beispiel |
|---|---|---|
| Temperatursensor | Reaktor, Ölbad, Gebinde | Temperaturzeitreihe, Messpunkt alle 5 Sekunden |
| Dosierpumpe | Rohstoff, Bindemittel, Weichmacher | Dosiermengenzeitreihe, Messpunkt alle 3 Sekunden |
| Ölbad | Temperiermedium (Öl) | Heizleistungs- und Umpumpmengenzeitreihe alle 10 Sekunden |
| Rührwerk | Reaktor | Drehmoment- und Umdrehungsgeschwindigkeitszeitreihe alle 2 Sekunden |

Weitere Vorbereitungen bestehen aus dem Aufbau eines vernetzten Computersystems. In diesem werden die Labormessgeräte und Laborobjekte mit eigener Aktivität sowie vorzugsweise elektronische Identifikatoreinheiten in einem System verbunden. Hierbei können kabelgebundene analoge Schnittstellen, wie durch die elektrische Spannung auswertbare, aber bevorzugt digitale Schnittstellen verwendet werden. Digitale Schnittstellen im Laborsystem sind dabei serielle Schnittstellen (z.B. RS232, RS485), USB-Schnittstellen und dergleichen. Auch Funktechniken, die eine kabellose digitale Verbindung möglich machen können verwendet werden, wie z.B. WiFi/WLAN nach den IEEE 802 Standards, Bluetooth, insbesondere Bluetooth Low Energy "BLE", die langgestrecken Funkstandards 5G, LTE 4G, LoRa, LTE-M, Sigfox oder NB-IoT. Digitale Schnittstellen können verschiedenen Protokolle verwenden, wobei erfindungsgemäß hier keine spezifischen Datenprotokolle festgelegt werden.

Laborobjekte ohne zusätzliche eigene elektronische Aktivität (wie z.B. Gebinde, Glasgefäße, Kolben, Rührer, Destillationskolonnen) können in den Vorbereitungen mit einem Tag bzw. Barcode versehen werden. Der Experimentator nutzt bevorzugt eine elektronische Identifikatoreinheit, um durch das Lesen des Tags bzw. Barcodes die Laborobjekte zu identifizieren und zu registrieren.

Bestimmungsgemäße Verwendungen (z.B. ein Labormessgerät in Kombination mit einer Messmethode oder eines Messbereiches), Ereignisse (z.B. Beginn und Ende einer Reaktion), Beobachtungen (z.B. Kategorien der Trübung) können ebenfalls durch einen Tag oder Barcode repräsentiert werden, der sich bevorzugt an einem leicht für den Experimentator zugänglichen Ort, wie z.B. auf einer Wand, im oder am Laborabzug und/oder in einem Laborbuch/Kladde befindet. Weiterhin können Tags oder Barcodes auch in computergestützten Menüs, Dashboard, Touchdisplays dargestellt und auswählbar sein. Bevorzugt nutzt der Experimentator eine elektronische Identifikatoreinheit, um durch das Lesen des Tags bzw. Barcodes oder alternativ durch Bedienung von Menüs, Dashboards, Touchdisplays die bestimmungsgemäße Verwendungen, Ereignisse und Beobachtungen zu identifizieren und zu dokumentieren. Dabei wird der Zeitpunkt in Form eines Zeitstempels protokolliert oder im zentralen Computersystem bei Eingang der Information erzeugt.

Eine besonders bevorzugte Ausführungsform des erfindungsgemäßen Verfahrens betrifft die Nutzung der erhobenen Daten und deren Vergleich mit bereits vorliegenden Daten früher durchgeführter Versuche. So ist z.B. eine Vorhersage von Versuchsverläufen, die dem Experimentator auf unsichere, aber auch mögliche zielgerichtetere Experimentdurchführung und geeignete Prozessparametereinstellungen hinweisen, möglich. An einem Dashboard/Bedienmenü kann der Mitarbeiter den Vergleich zu einem oder mehreren früheren Versuche auswählen und direkt mit seinen aktuellen Daten vergleichen lassen. So kann beispielsweise eine absehbare hohe Exothermie, eine Gelierung einer Reaktionsmischung ab einem kalkulierten Reaktionsumsatz, die zeitgenaue Abpassung von Versuchsschritten und/oder Prozessparametern wie Temperaturen, Drucke, Dosierraten, Exothermie- oder Viskositätsverläufe, aber auch Nebenkomponentenbildung aus z.B. spektroskopischer in-Prozessanalytik angezeigt werden. Hierbei ist es dann dem Experimentator möglich, diese Informationen direkt in der aktuellen Versuchdurchführung zu berücksichtigen und gezielt die Prozessführung anzupassen bzw. zu korrigieren. Derartige Anpassungen sind in Prozessleitsystemen ebenfalls üblich, basieren aber auf Steuerungsparameter, die die Einhaltung von konkreten einzelnen Prozessparametern erlauben (z.B. eine Temperaturregulierung eines Heizbades, Vakuumkontrolle einer Vakuumpumpe usw.) und erfordern große Investitionen in Pilotanlagentechnik und umfangreiche, teure Sicherheitsbetrachtungen. Im Gegensatz hierzu ist es hier nun dem Experimentator gemäß dieser vorteilhaften Ausgestaltung der Erfindung möglich, umfassendere Informationen und zeitnahe Anleitungen zum Handeln zu erhalten, um z.B. die Reproduzierbarkeit eines Versuches bereits im Forschungslabor zu verbessern, oder auch ganz bewusst Abweichungen zu prüfen und dabei zeitabhängige Prozessparameter genauer und effizienter in der Versuchsdurchführung zu berücksichtigen. Die Darstellung und der Vergleich von Ist- mit Sollwerten stellt somit eine aktive Regulierung dar, die Arbeit eines Experimentators zielgerichtet unterstützt und steuert.

Eine besonders bevorzugte Ausgestaltung des erfindungsgemäßen Verfahrens betrifft den Vergleich der Versuchsdurchführung im Forschungslabor auf produktionsrelevante Versuchsparameter. So wird im industriellen Skalierungsprozess mit jedem Skalierungsschritt zum Beispiel die Reaktorgröße, die Reaktorgeometrie, das Rührdesign etc. geändert. Derartige bereits absehbare, zukünftig umzusetzende Prozessänderungen - bedingt durch die gegebene Produktionsassetstruktur - können bereits in einer aktiven Vorhersage dieser Einflüsse dem Bediener zum Beispiel im Dashboard angezeigt werden, so dass produktionsrelevante Prozessbedingungen bereits in der Forschung im Syntheseverlauf gewählt werden können.

Ein weiterer Aspekt der vorliegenden Erfindung betrifft ein System zur Datenverarbeitung, umfassend Mittel zur Ausführung des Verfahrens nach einem der Ansprüche 1 bis 9. Für die Vorteile des erfindungsgemäßen Systems gelten die vorstehenden Vorteile des Verfahrens in analoger Weise.

Ein weiterer Aspekt der vorliegenden Erfindung betrifft ein Computerprogramm, umfassend Befehle, die bei der Ausführung des Programms durch einen Computer diesen veranlassen, das Verfahren/die Schritte des Verfahrens nach einem der Ansprüche 1 bis 9 auszuführen.

### Beispiel 1

Im Folgenden ist ein Beispiel beschrieben, dass das erfindungsgemäße comuterimplementierte Verfahren zur elektronischen Aufzeichnung einer Synthese als einem naturwissenschaftlichen Experiment beschreibt. Im Rahmen dieses Beispieles zeigen:
- Fig. 1 a-g: Schritte eines computerimplementierten Verfahrens zur elektronischen Aufzeichnung eines naturwissenschaftlichen Experimentes in einem elektronischen Laborjournal in schematisch, tabellarischer Darstellung,
- Fig. 2: die schematische Darstellung einer Hardwareimplementierung des computerimplementierten Verfahren gemäß Fig. 1 und
- Fig. 3: eine weitere schematische Darstellung des computerimplementierten Verfahrens zur elektronischen Aufzeichnung eines naturwissenschaftlichen Experimentes in einem elektronischen Laborjournal.

Bei dem computerimplementierten Verfahren zur elektronischen Aufzeichnung eines naturwissenschaftlichen Experimentes werden in einem Vorbereitungschritt die einzusetzenden Laborobjekte und Labormessgeräte festgelegt. Tabelle 3 zeigt die Laborobjekte und Labormessgeräte sowie deren Identifikationsbezeichung und die Identifikationskennung der verwendeten Objekte. Zudem ist als Ereignis das "Laborversuch Ende" gelistet.

**Tabelle 3: Festlegung der Labormessgeräte und Laborobjekte des Beispiels 1**

| **Laborobjekt, Labormessgeräte, Ereignisse** | **Identifikationsbezeichung** | **Identifikationskennung** |
|---|---|---|
| Laborjournal-Experimentkennung | LaborjounalID | ABC1234 |
| Identifikator | Identifikator ID | 0001, 0002 |
| Reaktor | ReaktorgefäßID | R003 |
| Waage | WaageID | WO09 |
| Temperatursensor | TempSensorID | T002 |
| Chemikalie | ChemikalieID | C12543, C80001 |
| Überkopfwaage | ÜberkopfwaageID | ÜWO010 |
| Gebinde | GebindeID | G7653 |
| Produkt | ProduktID | LaborjournalID |
| Laborversuch Ende | LaborversuchEnde | LabEnde |

In Tabelle 4 ist nun für die verwendeten weiteren Verknüpfungsregeln die bestimmungsgemäße Verwendung der Laborobjekte und Labormessgeräte aus Tabelle 3 auf andere Objekte als Variante der bestimmungsgemäßen Verwendung beschrieben und damit durch den Experimentator auswählbar.

**Tabelle 4: Verknüpfungsregel der Labormessgeräte, Laborobjekte und Ereignisse aus Tabelle 3**

| **Laborobjekt, Labormessgerät, Ereignis** | **Beschreibung** | **Angewendete Verknüpfungsregel** |
|---|---|---|
| Laborjournal-Experimentkennung | Name eines Experiments in einem Laborjournals | Wird über die IdentifikatorID verknüpft |
| Identifikator | Zuordnung zu einer Laborjournal-Experimentkennung | Dient dem Eintragen von Messgeräten, Laborobjekten und Ereignissen in die Verknüpfungstabelle |
| Reaktor | Bezeichnung eines Reaktors | Keine |
| Waage | Bezeichnung einer Waage | Bestimmungsgemäße Anwendung auf |
| | | Chemikalie, Reaktor und Gebinde |
| Temperatursensor | Bezeichnung eines Temperatursensors | Bestimmungsgemäße Anwendung auf Reaktor |
| Chemikalie | Bezeichnung eines Rohstoffes | keine |
| Überkopfwaage | Kontinuierliches Wiegen von eindosierten Chemikalien | Bestimmungsgemäße Anwendung auf Chemikalie |
| Gebinde | Abfüllung von Produkt | Bestimmungsgemäße Anwendung auf Produkt |
| Produkt | Produkt bezeichnung | Bestimmungsgemäße Anwendung auf Gebinde |
| Laborversuch Ende | Ereignisbezeichnung | Bestimmungsgemäße Anwendung auf Laborjournal- Kennung |

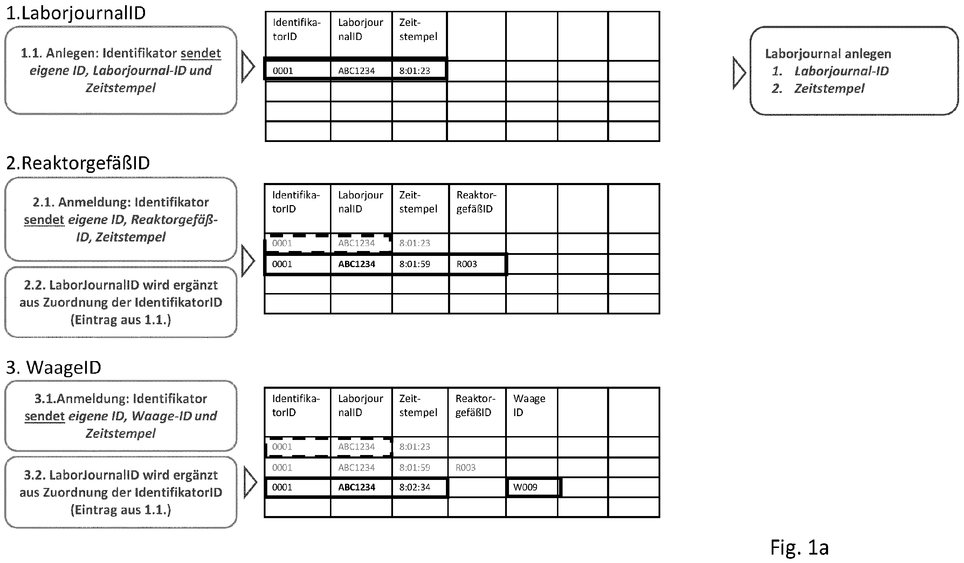

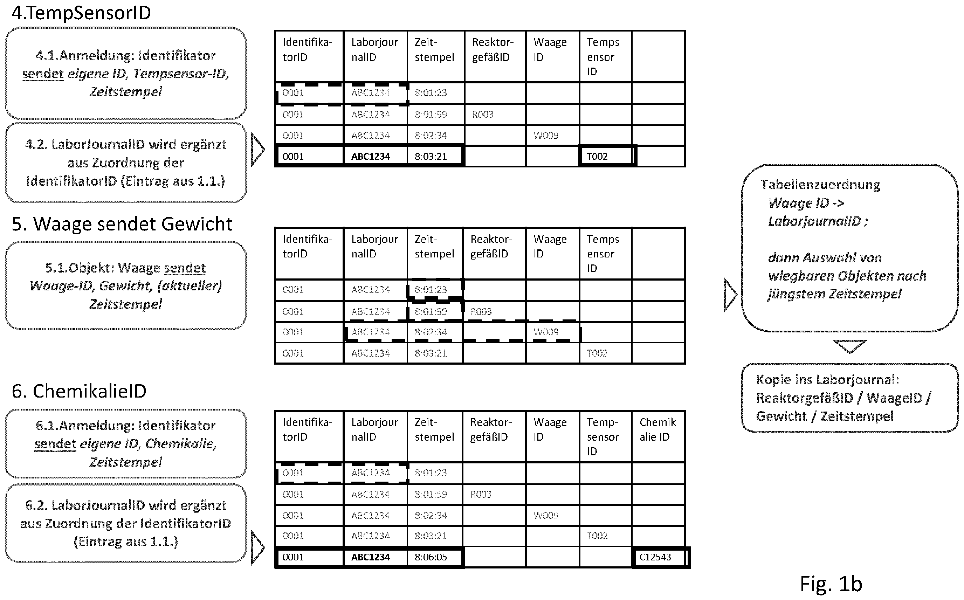

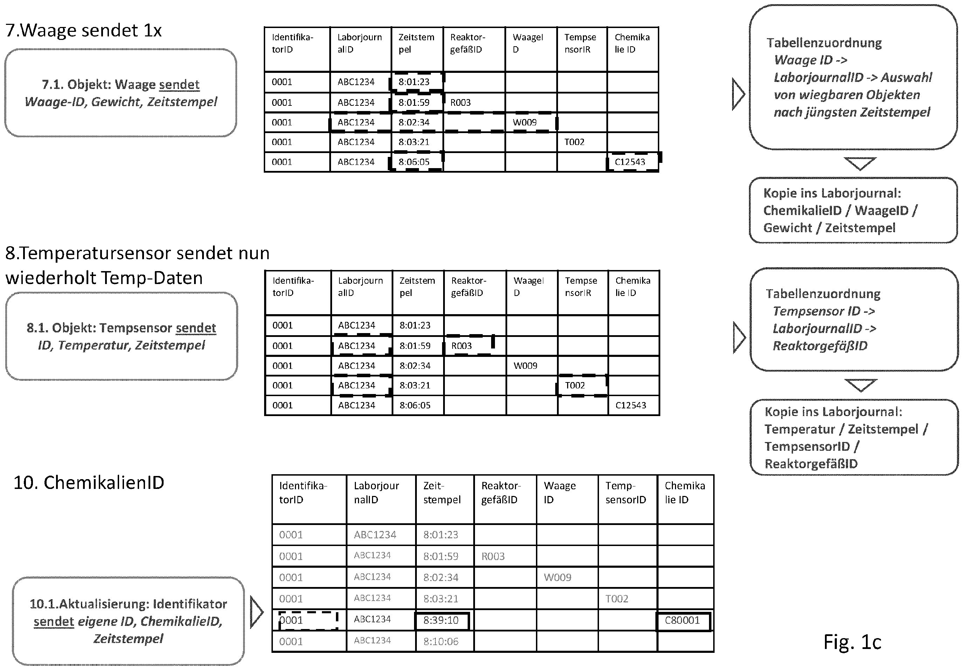

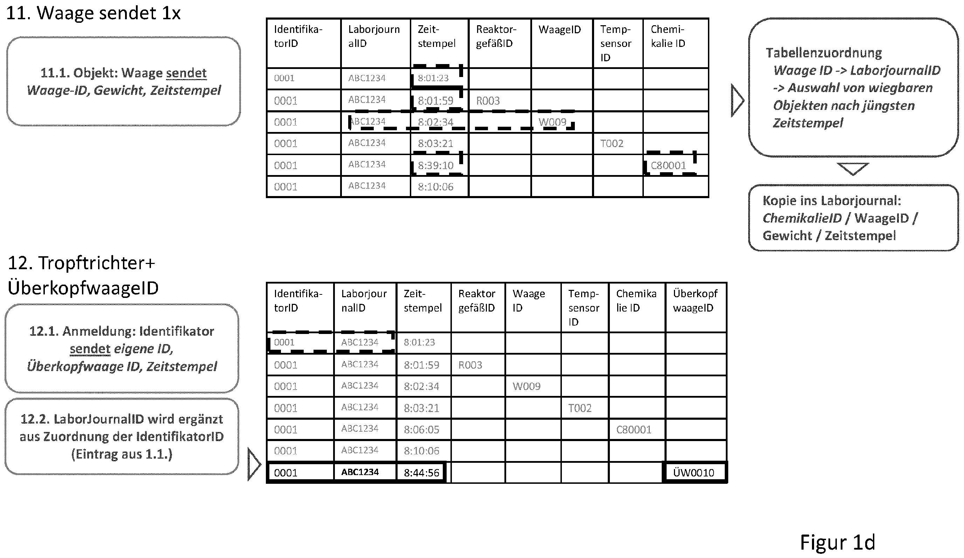

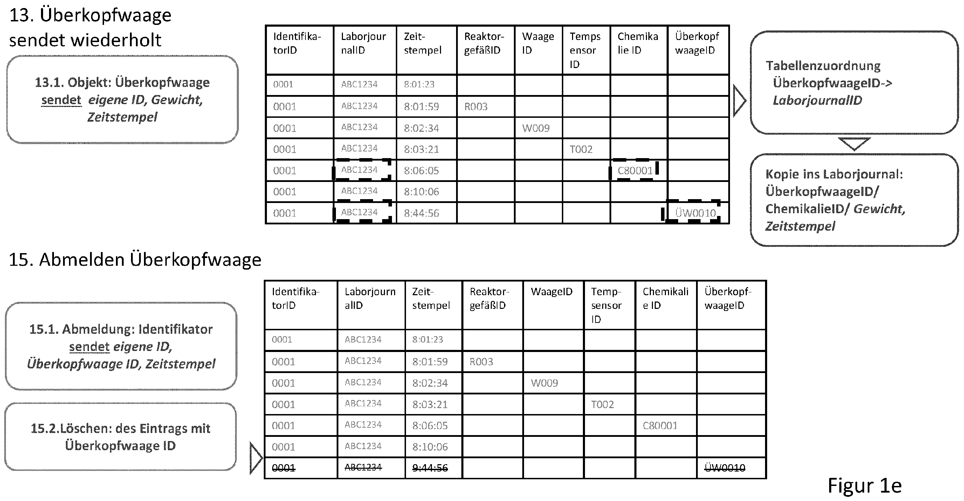

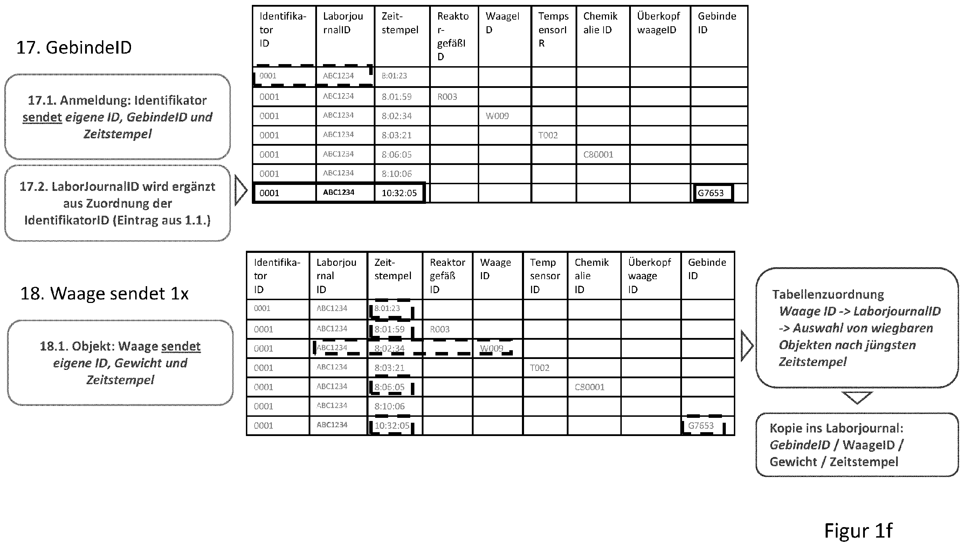

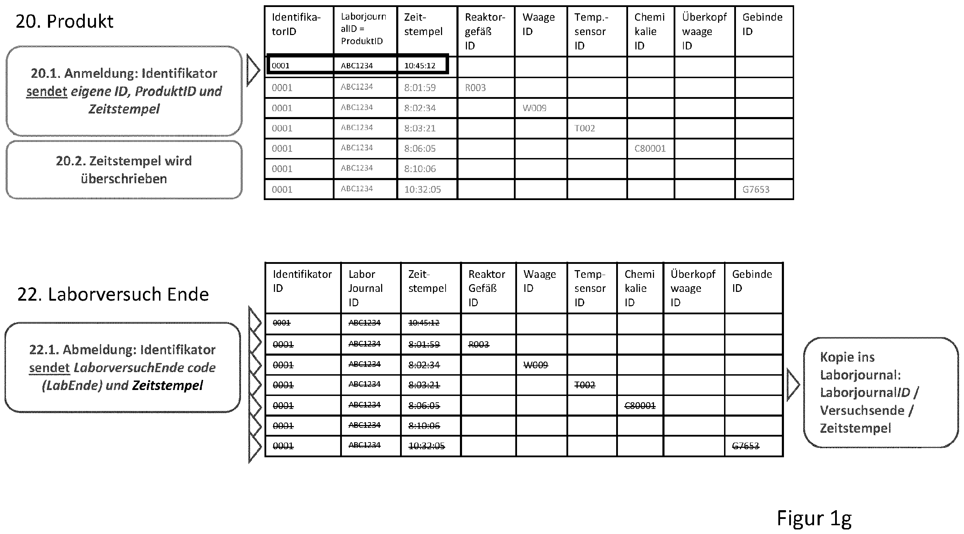

Im Folgenden sind nun die Arbeitsschritte in der Durchführung des erfindungsgemäßen Beispiels 1 in 22 Schritten beschrieben. Die einzelnen Schritte sind in den Fig. 1a bis 1g in tabellarischer Form dargestellt.
1. Experimentator nimmt einen Scanner (Identifikator) und scannt einen Laborjournaleintrag (siehe Schritt 1.1 in Fig 1a).
2. Experimentator nimmt einen 3-Hals-Kolben und scannt dessen ReaktorgefäßID (siehe Schritt 2.1 in Fig 1a).
3. Experimentator geht zu einer Waage und scannt deren WaagenID (siehe Schritt 3.1 in Fig 1a).
4. Experimentator nimmt einen Temperatursensor und scannt dessen TempsensorID (siehe Schritt 4.1 in Fig 1b).
5. Experimentator drückt die Tara-Funktion der Waage und stellt danach den 3-Hals-Kolben auf die Waage. Nach Gewichtskonstanz betätigt dieser eine Waagentaste (siehe Schritt 5.1 in Fig 1b, einmaliges Senden eines Gewichts von der Waage).
6. Experimentator nimmt eine Chemikalie und scannt deren ChemikalienID (siehe Schritt 6.1 in Fig 1b)
7. Experimentator drückt die Tara-Funktion der Waage und wiegt die im Synthesekonzept angegebene Menge der Chemikalie ein. Nach Gewichtskonstanz betätigt dieser erneut die Waagentaste (Schritt 7.1 in Fig 1c, einmaliges Senden eines Gewichts von der Waage).
8. Experimentator montiert den 3-Hals-Kolben mit Schraubzwinge an ein Gestell. Er setzt den Temperatursensor ein und schaltet diesen an (mit Schritt 8.1 in Fig 1c beginnt das kontinuierliche Senden von Temperaturdaten).
9. Experimentator nimmt ein Ölbad, stellt als Zieltemperatur 80°C ein und fährt dieses an den 3-Hals-Kolben heran bis der Kolben zu 60% eingetaucht ist.
10. Experimentator nimmt ein weiteres Chemikaliengebinde und scannt dessen ChemikalienID (siehe Schritt 10.1 in Fig 1c)
11. Experimentator nimmt einen Tropftrichter, stellt diesen mit Hilfe eines Korkrings auf die Waage, drückt die Tara-Funktion der Waage und wiegt die im Synthesekonzept angegebene Menge der zweiten Chemikalie ein. Nach Gewichtskonstanz betätigt dieser erneut die Waagentaste. (siehe Schritt 11.1 in Fig 1d)
12. Experimentator montiert den Tropftrichter hängend an die Überkopfwaage und scannt die ÜberkopfwaagenID der Überkopfwaage (siehe Schritt 12.1 in Fig 1d).
13. Experimentator schaltet die Überkopfwaage ein (mit Schritt 13.1 beginnt die Überkopfwaage kontinuierlich Gewichtsdaten zu senden - siehe in Fig 1e).
14. Nachdem das Thermometer 80°C anzeigt beginnt der Experimentator mit der Zudosierung. Dabei hält er den vorgegebenen Temperaturzielbereich ein, so dass die Temperatur nicht über 85°C steigt.
15. Nach vollständiger Dosierung der Chemikalie aus dem Tropftrichter in den Reaktor, lässt der Experimentator eine Stunde bei 80°C weiterrühren. Danach scannt er erneut die Überkopfwaage und meldet diese damit ab (siehe Schritt 15.1 in Fig 1e, damit endet auch das kontinuierliche Senden der Gewichtsdaten der Überkopfwaage aus Schritt 13.1.)
16. Experimentator erkennt, dass die geplante Reaktionszeit erreicht wurde und kühlt Reaktor ab, indem er das Ölbad herunterfährt.
17. Experimentator scannt GebindeID eine Gebindes zum Abfüllen des Syntheseproduktes, (siehe Schritt 17.1 in Fig 1f)
18. Experimentator drückt die Tara-Funktion der Waage und stellt danach das Gebinde auf die Waage. Nach Gewichtskonstanz betätigt dieser einer Waagentaste. Danach betätigt der Experimentator erneut die Tara-Taste.
19. Nach dem vollständigem Abkühlen des Reaktors wird dieser vom anhaftenden Öl des Ölbads gereinigt, Tropftrichter und Rührer abgebaut und das Syntheseprodukt in das Gebinde abgefüllt. Rührwerk, Temperatursensor werden abgeschaltet.
20. Experimentator scannt Produktbarcode
21. Experimentator stellt das Gebinde erneut auf die Waage. Nach Gewichtskonstanz betätigt dieser eine Waagentaste.
22. Experimentator scannt Barcode "Laborversuch Ende" (siehe Schritt 22.1 in Fig 1g)

Die Fig. 1a bis 1g zeigen zudem für jeden entsprechenden Schritt eine Visualisierung der Aktionen des Computersystems. Auf der linken Seite sind jeweils die elektronischen Aktionen der Identifikatoreinheit sowie der Labormessgeräte und Laborobjekte mit elektronischer Funktion als Eingangssignal beschrieben. In der Mitte ist eine tabellarische Darstellung ("temporäre Verknüpfungstabelle") gezeigt, die die bereits registrierten Laborobjekte, Labormessgeräte und Chemikalien und die Anwendung der Verknüpfungsregeln zeigt. Auf der rechten Seite sind die Komponenten der im Laborjournal abgespeicherten Daten-Reihen aufgeführt. Die tabellarische Darstellung der Verknüpfungsregeln ist eine illustrative Darstellung der Umsetzung der Aktionen des Computersystems und es versteht sich, dass hiermit nicht die computertechnische Umsetzung der Erfindung beschränkt wird.

Die Figuren 1a bis 1g zeigen die Arbeitsschritte, die sich unterteilen lassen in a) (links und Mitte) Anlegen einer Laborjournal-ID, b) (links und Mitte) Registrieren eines Laborobjektes oder eines Labormessgeräts oder einer Chemikalie durch die Identifikatoreinheit und Erzeugen eines Eintrages in der Verknüpfungstabelle (Mitte), c) Senden einer Nachricht von einem Laborobjekt oder einem Labormessgerät an das Computersystem (illustriert durch einen Pfeil, der auf die entsprechende Zeile der Verknüpfungstabelle weist) mit anschließender Auswertung der Verknüpfungstabelle (Mitte und rechts) unter Anwendung der Verknüpfungsregel (siehe auch Tabelle 4) und Übertrag der Daten-Reihe (mit Angabe seiner Komponenten) an ein elektronisches Laborjournal (rechts), d) Senden einer Nachricht über ein Ereignis durch die Identifikatoreinheit (links) und e) Abmeldung eines Objektes und Löschen des Eintrages in der Verknüpfungstabelle (Mitte).

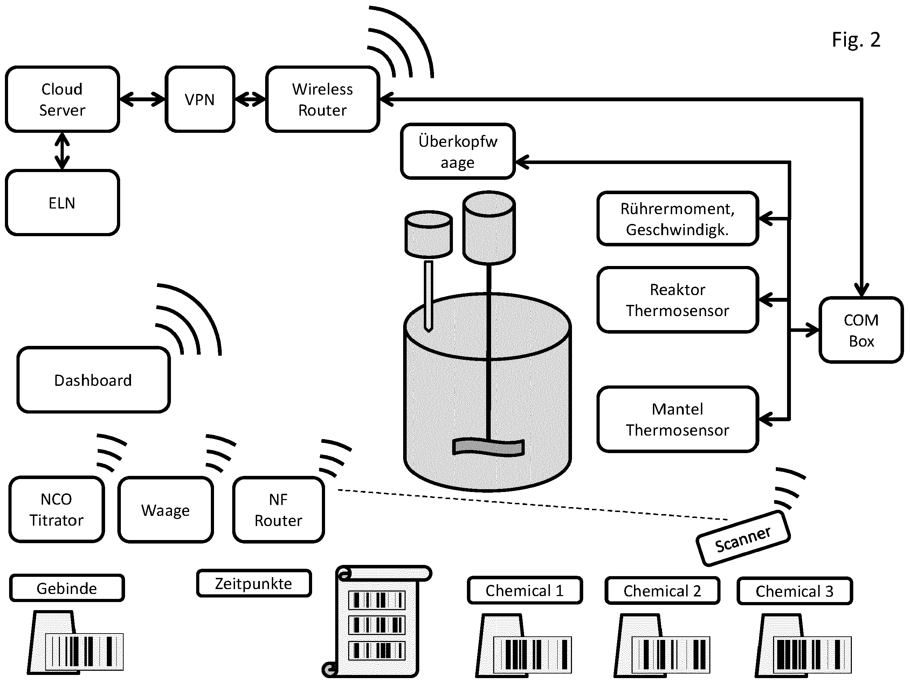

Neueinträge in die Verknüpfungstabelle sind zur Erleichterung der Lesbarkeit der Visualierung in Fig. 1a-1g in der Tabelle durchgehend umrandet. Abfragen der Verknüpfungstabelle sind gestrichelt umrandet. Nach Anmeldung/Registrierung eines Objektes erfolgt automatisch eine Recherche in der Verknüpfungstabelle (siehe gestrichelt umrandete Felder z.B. in Fig. 1a 2. "Reaktorgefäß-ID"), um Einträge mit der gleichen IdentifikatorID zu suchen. Wenn dies erfolgreich (also ein solcher Eintrag vorhanden) ist, wird die zugehörige LaborjournalID in dem neuen Eintrag nachgetragen. So wird in Fig. 1a, 2. "Reaktorgefäß-ID" zunächst ermittelt, dass der Identifikator mit der Identifikator-ID _{"}0001" bereits mit dem Laborjournal mit der Laborjournal-ID _{"}ABC1234" verknüpft ist. Damit wird der Reaktor mit der Reaktorgefäß-ID _{"}R003" mit der Laborjournal-ID _{"}ABC1234" ebenfalls verknüpft.

Auf der rechten Seite der Fig. 1a-1g findet sich eine Beschreibung der erzeugten Verknüpfungen, die in der Verknüpfungstabelle (Mitte) recherchiert werden und dann als Daten-Reihe in dem elektronischen Laborjournal abgespeichert wird. Die somit erhaltene zeitliche Sequenz der Daten-Reihen des erfindungsgemäßen Beispiels 1 sind in Tabelle 5, die das elektronische Laborjournal visualisiert, gezeigt. Die Daten-Reihen des Beispiels 1 haben somit 10 verschiedene Komponenten.

Aus Übersichtsgründen sind in Tabelle 5 zeitliche Abschnitte nicht im Detail gezeigt, in denen analoge weitere Daten-Reihen des Temperatursensors T002 und der Überkopfwaage ÜWO010 erzeugt wurden. Diese Daten-Reihen werden in Tabelle 5 durch eine Zeile, die mit dem Kürzel _{"}[...]" beginnt, repräsentiert. Die Tabelle 5 stellt die Datenvektoren des Experiments mit der VersuchsID ABC1234 im elektronischen Laborjournal dar, die gemäß dem erfindungsgemäßen Verfahren erzeugt wurden. Tabelle 5 zeigt die Daten-Reihen, die durch die Schritte 5, 7, 8, 11, 13, 18, 21 und 22 erhalten wurden (siehe auch Kommentarspalte in Tabelle 5).

Fig. 2 zeigt eine Skizze der Implementierung einer Hardwarekonfiguration des erfindungsgemäßen Verfahrens. Dabei ist ein chemischer Reaktor mit verschiedenen Sensoren versehen, die es erlauben, die Rührerparameter, Temperaturen im Reaktor als auch in einem Heizmantel (bzw. Heizbad) sowie gravimetrische Dosierwerte zu erheben. Es ist in der Praxis von Vorteil, solche Messsensoren über elektrische Kabel anzuschließen, z.B. über einen COM (Communication) Port. In der Industrie üblicherweise verfügbare Anschlüsse sind serielle Anschlüsse, wie z.B. RS232, RS 485, USB oder dergleichen. Der COM-Port ist als eigene Einheit ausgeführt und wird in dem Beispiel an einen Router mittels LAN (Local Area Network-Technik) angeschlossen. Der Router verbindet kabellos über WLAN (Wireless Local Area Network.Technik) weitere Geräte: Fig. 2 zeigt hier den Anschluss an eine Waage, einen Isocyanat (NCO) Titrator und an ein NF (Near Field Communication) Gerät. Für letzteres stehen verschiedene Technologien zur Auswahl, besonders geeignet für dieses Beispiel ist BLE (Bluetooth Low Energy). In diesem Ausführungsbeispiel wird die NF Technologie insbesondere zum Anschluß von Barcodescannern genutzt. Der Barcodescanner wird hier als elektronischer Identifikator für Chemikalien und Gebinde verwendet.

Der Router als zentrale digitale Einheit im chemischen Labor ist über eine gesicherte Verbindung (VPN - Virtual Private Network) mit einem Server verbunden. Derartige Server werden von Dienstleistern weltweit angeboten (Cloud Computing Services). Dies hat den Vorteil, dass eine gesicherte, verschlüsselte Kommunikation zwischen den üblicherweise dezentral lokalisierten chemischen Laboren und der IT Serverinfrastruktur sichergestellt ist. Auf dem Server werden die Daten, wie bereits beschrieben, verarbeitet und zu Daten-Reihen in einem elektronischen Laborjournal zusammengeführt. Weiterhin erfolgt ein aktives Feedback an den Labormitarbeiter über einen Bildschirm (_{"}Dashboard"), das die erhobenen Messwerte in Kontext setzt und Empfehlungen oder Vergleiche zu früheren Versuchen, bzw. Scale-Up relevante Parameter anzeigt. Das Dashboard wird dabei von dem Router ebenfalls kabelfrei angesteuert, z.B. durch Datenprotokolle, die mit einem üblichen http-fähigen Browser anzeigbar sind. Dies hat den Vorteil, dass das Dashboard z.B. in Form mobiler Endgeräte wie Smartphones und/oder Tablets auch im Laboralltag leicht mitgeführt werden kann. Auch können standordfeste Laborcomputer derartige Informationen zusätzlich anzeigen.

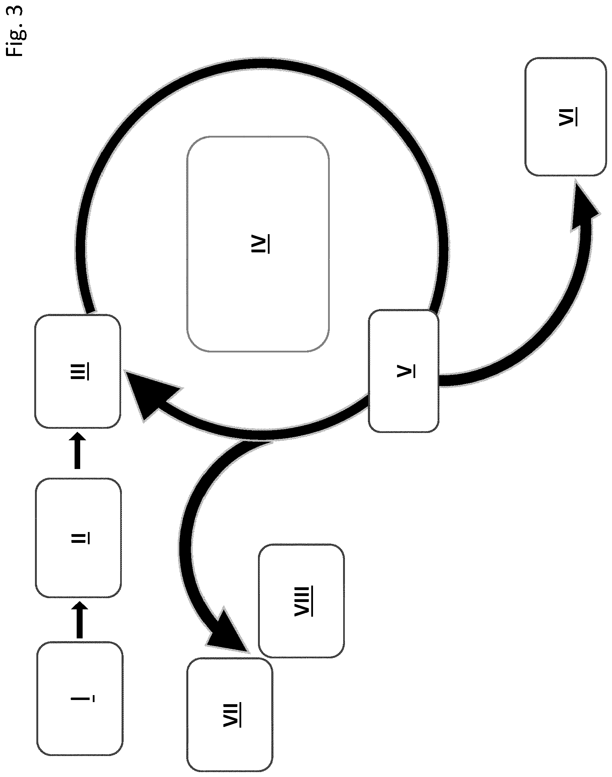

Fig. 3 zeigt in einer weiteren schematischen Darstellung die Schritte eines computerimplementierten Verfahrens zur elektronischen Aufzeichnung eines naturwissenschaftlichen Experiments in einem elektronischen Laborjournal. In einem ersten Planungs- und Vorbereitungsschritt (Schritt I) erfolgt die Bereitstellung eines vernetzten Computersystems. Ferner sorgt der Experimentator für die Bereitstellung von Chemikalien sowie Laborobjekten und/oder Labormessgeräten, soweit diese zur Durchführung des naturwissenschaftlichen Experimentes benötigt werden. Ferner plant er deren bestimmungsgemäße Verwendung. So kann beispielsweise ein Temperatursensor zur Messung der Temperatur des Inhalts eines Reaktorgefäßes eingesetzt werden.

Bei dem Comutersystem kann es sich um einen entsprechend programmierten Stanndardlaborrechner handeln, welcher mit entsprechenden Schnittstellen ausgerüstet ist, die ihrerseits beispielsweise mit den Labormessgeräten verbunden sind.

Sodann wird in einem Schritt II eine Laborjournal-ID mittels einer elektronischen Identifikatoreinheit, kurz _{"}Identifikator", beispielsweise durch einlesen eines Balken- oder QR-Codes angelegt.

Anschließend erfolgt in Schritt III die Registrierung der im Experiment zu verwendenden Laborobjekte (beispielsweise Reaktorgefäße, Heiteinrichtungen, Titratoren etc.), Labormessgeräte (z.B. Temperatursensor, Waage etc.) und Chemikalien und deren Verknüpfung mit der zuvor angelegten Laborjournal-ID mittels der elektronischen Indentifikatoreinheit.

Im Verlauf der Durchführung des Experiments - hier als Schritt IV in einem kreisförmigen Pfeil dargestellt - werden nun die Laborobjekte, Labormessgeräte und Chemikalien bestimmungsgemäß verwendet. Dabei werden Ereignisse und/oder Beobachtungen zu der/den Chemikalie(n) (z.B. Trübung der Chemikalie) und/oder zu den Laborobjekten (z.B. Start einer Temperaturmessreihe) und/oder Messergebnisse der Labormessgeräte (z.B. Gewichts- oder Temperaturmessungsdaten), wie in Beispiel 1 im Detail beschrieben, unter Anwendung der Verknüpfung mit der Laborjournal-Experimentkennung und optional weiterer Verknüpfungsregeln an das Computersystem übermittelt (Schritt V). Dabei erzeugt das Computersystem in der in Beispiel 1 beschriebenen Weise Daten-Reihen, welche in dem elektronischen Laborjournal abgelegt werden (Schritt VI).

Im Experiment nicht mehr benötigte Laborobjekte oder Labormessgeräte können durch den Identifikator einzeln abgemeldet und, wie in der Grafik der Fig. 3 dargestellt, entsprechend aus dem Aktivitätszyklus der Experimentdurchführung ausgeleitet werden (Schritt VII). Nach Beendigung des gesamten Versuches werden sämtliche verwendeten Laborobjekte, Labormessgeräte und Chemikalien durch den Identifikator abgemeldet (Schritt VIII) und der Versuch beendet.

Wie aus Beispiel 1 deutlich wird, erlaubt das erfindungsgemäße Verfahren eine umfassende, präzise Dokumentation des naturwissenschaftlichen Experiments. Insbesondere wird dem Experimentator ermöglicht, mit nur geringem eigenem Aufwand die Versuchsdurchführung zu dokumentieren. Hierbei wird die notwendige Freiheit einer variablen Versuchsdurchführung nicht eingeschränkt, da der Experimentator jederzeit seine Experimentdurchführung ändern kann und eine entsprechenden Dokumentation nach dem erfindungsgemäßen Verfahren weiterhin flexibel erzeugt wird. Der Umfang der Dokumentation und die Detailtiefe wird durch den Umfang der definierten Laborobjekte, Chemikalien, Labormessgeräte, Ereignisse und Beobachtungen sowie deren Verknüpfungsregeln definiert. Um eine präzise Dokumentation zu ermöglichen, ist eine zusätzliche parallele Kommunikation zwischen Experimentplaner und Experimentator nicht mehr nötig, da umfassend protokolliert wird, wie das in Tabelle 5 exemplarisch dagestellte Laborjournal zeigt. Möglich ist ebenfalls, dass eine wirtschaftliche, gemeinschaftliche Nutzung von Laborressourcen zwischen mehreren Experimentatoren erfolgt. Die Daten-Reihen, die gemäß des erfindungsgemäßen Verfahrens erzeugt werden, erlauben eine Auswertung mit moderner Datentechnik, ohne eine aufwändige Datenkurierung anwenden zu müssen.

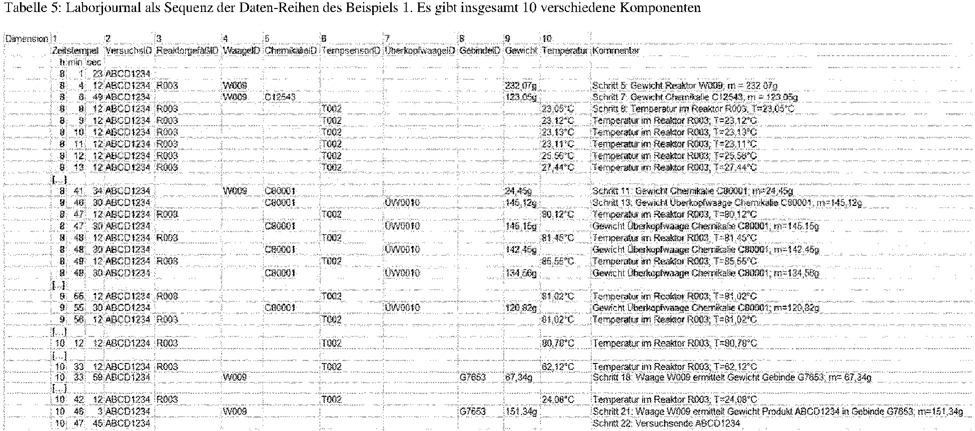

## Patentansprüche

1. Computerimplementiertes Verfahren zur elektronischen Aufzeichnung von naturwissenschaftlichen Experimenten in einem elektronischen Laborjournal, **gekennzeichnet durch** folgende Verfahrensschritte:
- Bereitstellung wenigstens einer Chemikalie sowie wenigstens eines Laborobjektes und/oder wenigstens eines Labormessgerätes zur Durchführung eines naturwissenschaftlichen Experimentes,
- Registrierung der wenigstens einen Chemikalie sowie des wenigstens einen Laborobjektes und/oder des wenigstens einen Labormessgerätes zumindest für die Zeit ihrer/seiner Verwendung in dem naturwissenschaftlichen Experiment und Verknüpfung mit einer Laborjournal-Experimentkennung jeweils in einem Computersystem,
- Übermittlung wenigstens eines Ereignisses und/oder wenigstens einer Beobachtung zu der wenigstens einen Chemikalie und /oder zu dem wenigstens einen Laborobjekt und/oder wenigstens eines Messergebnisses des wenigstens einen Labormessgerätes an das Computersystem jeweils zusammen mit einem Zeitstempel unter Anwendung der Verknüpfung mit der Laborjournal-Experimentkennung und optional weiterer Verknüpfungsregeln, wobei das Computersystem wenigstens eine Daten-Reihe erzeugt und in einem elektronischen Laborjournal abspeichert.

2. Verfahren nach Anspruch 1,
**dadurch gekennzeichnet, dass**
die Registrierung der wenigstens einen Chemikalie, des wenigstens einen Laborobjektes und/oder des wenigstens einen Labormessgerätes und die Verknüpfung mit einer Laborjournal-Experimentkennung mittels einer elektronischen Identifikatoreinheit erfolgt.

3. Verfahren nach Anspruch 1 oder 2,
**dadurch gekennzeichnet, dass**
die Übermittlung des wenigstens einen Ereignisses und/oder der wenigstens einen Beobachtung zu der wenigstens einen Chemikalie und /oder zu dem wenigstens einen Laborobjekt an das Computersystem mittels einer elektronischen Identifikatoreinheit erfolgt.

4. Verfahren nach Anspruch 2 oder 3,
**dadurch gekennzeichnet, dass**
die Identifikatoreinheit ausgebildet ist
- als optisches Lesegerät, bevorzugt als Kamera, speziell als Kamera mit QR-Scanner, oder als Barcode- oder QR-Codescanner, oder
- als akustisches Lesegerät oder Spracherkennungseinheit, oder
- als Funk-Leseeinheit, bevorzugt als RFID-Leseeinheit, insbesondere als auf dem NFC-Standard basierende RFID-Leseeinheit IPS, oder
- als Messeinheit zur Orstbestimmung.

5. Verfahren nach einem der Ansprüche 2 bis 4,
**dadurch gekennzeichnet, dass**
die Identifikatoreinheit als System verteilter Sensoren ausgebildet ist.

6. Verfahren nach einem der Ansprüche 1 bis 5,
**dadurch gekennzeichnet, dass**
das Computersystem weitere Verknüpfungsregeln anwendet, derart, dass die wenigstens eine Chemikalie, das wenigstens eine Laborobjekt und/oder das wenigstens eine Labormessgerät einander zugeordnet werden.

7. Verfahren nach Anspruch 6,
**dadurch gekennzeichnet, dass**
die Zuordnung auf einer
- bestimmungsgemäßen Verwendung des wenigstens einen Laborobjekts und/oder des wenigstens einen Labormessgeräts,
- einer zeitlichen Reihenfolge der Registrierung der wenigstens einen Chemikalie, des wenigstens einen Laborobjekts und/oder des wenigstens einen Labormessgeräts,
- der relativen räumlichen Position der wenigstens einen Chemikalie, des wenigstens einen Laborobjekts und/oder des wenigstens einen Labormessgeräts zueinander, oder
- einer Kombination der vorstehenden
basiert.

8. Verfahren nach einem der Ansprüche 1 bis 7,
**dadurch gekennzeichnet, dass**
das wenigstens eine Laborobjekt als Laborbereich ausgebildet ist, wobei das Verfahren bevorzugt in einer Mehrzahl von Laborbereichen durchgeführt wird.

9. Verfahren nach einem der Ansprüche 1 bis 8,
**dadurch gekennzeichnet, dass**
das wenigstens eine Laborobjekt als Analytikraum, Messraum, Chemikalienlager, Reinigungsplatz, Applikationsraum oder Logistikbereich ausgebildet ist

10. Verwendung des Verfahrens nach einem der Ansprüche 1 bis 9 für die Synthese, Formulierung, Herstellung und/oder Prüfung von Feinchemikalien, Reaktionsmischungen, Zwischenstufen und Präparationen, Reagenzien, technischen Rohstoffen und Formulierungen von Katalysatoren, Lacken, Klebstoffen, Gießharzrohstoffen, Schäumen, Thermoplasten, Kosmetik, Lebensmitteln, Wirkstoffen, Druckfarben, Pharmazeutika, Pflanzenschutzmitteln.

11. System zur Datenverarbeitung, umfassend Mittel zur Ausführung des Verfahrens nach einem der Ansprüche 1 bis 9.

12. Computerprogramm, umfassend Befehle, die bei der Ausführung des Programms durch einen Computer diesen veranlassen, das Verfahren/die Schritte des Verfahrens nach einem der Ansprüche 1 bis 9 auszuführen.
